# EUROPEAN PATENT SPECIFICATION

(11) **EP 4 251 220 B1**
(45) Date of publication and mention of the grant of the patent: **05.11.2025**
(21) Application number: 21819222.7
(22) Date of filing: 24.11.2021
(51) Int. Cl.: A61L 27/50, A61K 9/00, A61K 47/14, A61L 27/52, A61L 27/54, A61K 9/06

(54) **BIOCOMPATIBLE ORGANOGEL MATRICES FOR PREPARATION OF A DRUG DELIVERY DEPOT**
BIOKOMPATIBLE ORGANOGELMATRIZEN ZUR HERSTELLUNG EINES WIRKSTOFFFREISETZUNGSDEPOTS
MATRICES D'ORGANOGEL BIOCOMPATIBLES POUR LA PRÉPARATION D'UN DÉPÔT D'ADMINISTRATION DE MÉDICAMENT

(30) Priority: 25.11.2020 US 202063118139 P; 10.11.2021 US 202163277865 P
(43) Date of publication of application: 04.10.2023
(73) Proprietor: DePuy Synthes Products, Inc., Raynham, MA 02767 (US)
(72) Inventor: FLOREK, Charles, Chester, Pennsylvania 19380 (US); COZZONE, Eric, Glenmoore, Pennsylvania 19343 (US); ARMBRUSTER, David A., Chester, Pennsylvania 19380 (US)
(74) Representative: Carpmaels & Ransford LLP
(86) International application number: PCT/IB2021/060927
(87) International publication number: WO 2022/112967

(56) References cited:
- WO-A1-2020/212946
- US-A1- 2020 330 380
- ZINIC: "Cholesterol-based gelators", 1 January 2005 (2005-01-01), XP055890409, Retrieved from the Internet <URL:http://www.ncbi.nlm.nih.gov/pubmed/22160336> [retrieved on 20220210]
- GIOIA BRUNA ET AL: ""Green" Organogelators: Design and Applications", 6 February 2018 (2018-02-06), XP055890421, Retrieved from the Internet <URL:https://www.rroij.com/open-access/green-organogelators-design-and-applications.pdf> [retrieved on 20220210]

## Description

This invention was made with government support under grant no. W81XWH-20-1-0378 awarded by the U.S. Army Medical Research Acquisition Activity (USAMRAA). The government has certain rights in the invention.

### CROSS-REFERENCE TO RELATED APPLICATIONS

This application claims the benefit of U.S. Appl. No. 63/277,865, filed on Nov. 10, 2021, and U.S. Appl. No. 63/118,139, filed on Nov. 25, 2020.

U.S. Appl. No. 16/851,177, filed on April 17, 2020, claims the benefit of U.S. Provisional Application No. 62/835,556, filed on April 18, 2019.

### FIELD OF DISCLOSURE

The present invention is defined in the appended claims. The present disclosure is directed to the preparation of a local drug depot formed from an organogel matrix in a non-sterile environment, and the application thereof to a non-sterile open wound. The present disclosure is also directed to the perioperative and intraoperative preparation and delivery of organogel matrix drug delivery depots for local delivery of active agents to a surgical site or traumatic wound. More particularly, embodiments of the present disclosure are directed to preparation and local delivery of antimicrobial or anesthetic drug depots to a surgical site including one or more implantable medical devices, such as implantable orthopedic medical devices.

Certain surgical methods are described with reference to the composition of the present invention. Whilst no claim is directed to these methods per se, the composition is capable of being used and is intended to be used in such methods

### BACKGROUND

Foreign bodies, such as orthopedic implants, are a risk factor for postsurgical infection. References to antibiotic and antimicrobial eluting devices are plentiful in the literature, but commercially-available devices are rare. Bone cements, such as poly(methyl methacrylate) (PMMA) and calcium sulfate cements are used on and off label to deliver antibiotics to orthopedic surgical sites.

PMMA cement is non-resorbable and its use necessitates a removal operation. Additionally, the amount of PMMA needed for anti-infective therapy is especially disadvantageous in orthopedic applications due to limited soft tissue availability (i.e., limited volume for placement). Incomplete elution of antibiotics from PMMA cements results in uncertainty of dose. Furthermore, long-term low-dose delivery can lead to antibiotic resistance development. Additionally, the implanted PMMA material (e.g., beads) presents another foreign body for bacterial colonization and growth.

Calcium sulfate cement can be used as an antibiotic delivery reservoir in bone defects or in soft tissue surrounding an orthopedic surgical site. In the US, studies have shown that calcium sulfate-based antibiotic therapies fail to provide controlled release of antibiotics for more than 3 days.

Another existing infection treatment option used is a surgeon directly delivering powdered antibiotic into the surgical site. Direct application of vancomycin powder in spine surgery was effective in case series, and a 1000 patient clinical trial has been conducted to measure the effect of local delivery of vancomycin on deep surgical site infections (SSIs) in high-risk trauma surgery. Nevertheless, antibiotic powder application does not provide either sustained or controlled local tissue concentrations. Further, its use is limited to open surgical procedures, thus eliminating its treatment potential from percutaneous or minimally-invasive surgical procedures.

Hydrogels have also been considered as a delivery vehicle; however, their elution profiles are typically dominated by burst release with limited controlled, sustained release. Some examples include Novagenit's Defensive Antibacterial Coating (DAC) hydrogel, Dr. Reddy's laboratories" DFA-02, and Poloxamer 407 thermoreversible hydrogels. One study of Novagenit's DAC hyaluronan-poly-D,L-lactide hydrogel demonstrated that greater than 60% of vancomycin was released within the first 4 hours and that greater than 80% was released within 24 hours (Giavaresi G, Meani E, Sartori M, Ferrari, A, Bellini D, Sacchetta AC, Meraner J, Sambri A, Vocale C, Sambri V, Fini M, Romanó CL, International Orthopaedics (SCIOT) 2014; 38:1505-1512). A study of Dr. Reddy's DFA-02 gel reported results with a majority of antibiotic elution within 24 hours (Penn-Barwell JG, Murray CK, and Wenke JC, J Orthop Trauma 2014; 28:370-375). A study of Poloxamer 407 thermoreversible hydrogel demonstrated extended vancomycin release in vitro; however, the local vancomycin concentration in a rat model at 24 and 48 hours was only 6% and 0.6% of the concentration at 4 hours demonstrating a significant decrease from initial release rates (Veyries ML, Couarraze G, Geiger S, Agnely F, Massias L, Kunzli B, Faurisson F, Rouveix B, International Journal of Pharmaceutics 1999; 192:183-193).

WO 2020/212946 A1 is directed to an organogel drug depot for use in delivering an active agent to a surgical site, such as an implant site, for instance an orthopedic implant site. WO 2020/212946 A1 is also directed to an organogel drug depot for use in a non-sterile environment and application to a non-sterile open wound site. In a further embodiment of WO 2020/212946 A1, there is disclosed a system for preparing an organogel drug depot including an organogel matrix comprising an organogelator and a biocompatible organic solvent, an active agent comprising solid particles, a container including at least one wall having an outer surface and defining a volume capable of containing the organogel matrix and active agent solid particles, and a heating component configured to contact the outer surface and supply an amount of heat to the container. According to certain embodiments of WO 2020/212946 A1, the active agent includes an antimicrobial agent, antibiotic agent, or a local anesthetic agent, or combination of the aforementioned active agents. According to certain embodiments of WO 2020/212946 A1, the organogel matrix has a melting point above 37 °C. According to further embodiments of WO 2020/212946 A1, the biocompatible organic solvent can include a biocompatible oil derived from a plant or animal, or synthetic derivatives thereof.

Sustained local release of antibiotics without removal of a device can be achieved with a bioresorbable antibacterial coating on a medical device; however, antibiotic coated devices in the orthopedic segment offer unique challenges. Many part numbers are required to fit patient anatomy, resulting in logistical challenges in coating, storing, and delivering sufficient stock of each size before expiration. Antibacterial implants would require a duplication of the inventory of the analogous non-antibacterial devices. Furthermore, the repeated sterilization of graphic cases is prohibitive to biodegradable antibacterial coatings, so alternate logistics are required.

Some difficulties associated with coated medical devices includes the limited market size per regulatory clearance, the necessity of duplicating inventory, and the technical challenge of coating the extensive varieties of anatomic implant shapes. Coated medical devices do not permit the surgeon to select desired antibiotics or combination of antibiotics. Evaluation of patient-specific risk factors or the species and sensitivities of bacteria recovered from patient tissues are important criteria in selecting the desired antimicrobial agents and dosage.

### SUMMARY

Some difficul Accordingly, it would be beneficial to provide a drug depot that can be perioperatively or intraoperatively prepared and intraoperatively delivered to a surgical site, for instance a surgical site including one or more implantable medical devices, such as an implantable orthopedic medical device, where the drug depot is resistant to irrigation, resistant to migration from the surgical site and can provide controlled release of an active agent, such as an antimicrobial, antibiotic, or local anesthetic agent, or a combination thereof. In other words, the drug depot can remain at the surgical site for the duration of time necessary for the desired release of the active agent

In additional embodiments, it would be beneficial to provide a drug depot that can be contemporaneously prepared and delivered to a non-sterile open wound site in a non-surgical setting; (i.e., a non-sterile environment), where the drug depot is migration resistant and can provide controlled release of an active agent, such as an antimicrobial agent or a local anesthetic. Such a drug depot that can be contemporaneously prepared and delivered can have particular advantage for use in acute and/or traumatic emergency treatment settings with non-sterile open wounds involving significant soft and hard tissue damage, such as for use by emergency medical technicians or combat personnel, where the drug depot is contemporaneously prepared and delivered to the non-sterile open wound site. Such benefits include the ability to immediately deliver necessary anti-infective and pain relief treatment to a specific wound site of patient, where the drug depot is configured to remain at the site of delivery.

The rate of battlefield wound infection and amputation for the United States military is currently considered to be unacceptably high. The Tactical Combat Casualty Care guidelines recommend that an injured soldier receive a tourniquet to control bleeding and later receive either oral moxifloxacin or IV/IO/IM ertapenem sodium. These guidelines do not include point-of-care local infection treatment. Thus, there is a need for an antibacterial wound protectant that can be applied in non-sterile austere environments, including extreme temperature conditions, that can be locally applied to traumatized tissue, particularly deep tissue exposure, and is adherent to blood-contaminated tissue, and generates high local concentrations of battlefield-relevant antibiotic for at least 3 days for transport to definitive wound treatment locations. Accordingly, the present inventors have developed an antibacterial wound protectant with desired handling properties and *in vitro* antibiotic release characteristics for use in non-sterile austere environments.

The present invention is defined in the independent claim. The methods described here below are not claimed.

According to certain embodiments, the melting point peak is in the range of about 63° C to about 102° C. In certain further embodiments, the melting point peak is in the range of about 47° C to about 66° C.

According to certain embodiments, the organogel matrix defines a semi-solid state between the temperatures of about 0° C to about 70° C. In further embodiments, the organogel matrix defines a semi-solid state between the temperatures of 4° C to about 65° C.

The organogelator includes a mixture of cholesterol and at least one of hydrogenated castor oil or a mono- di- glyceride blend. The organogelator may further include mono- or di- glycerides having a chain length greater than c10 (an aliphatic carbon molecule having 10 carbon atoms or greater), hydrogenated oils, sodium salts of fatty acids, and combinations thereof.

According to certain embodiments, the organogelator comprises less than 50% by weight of the organogel matrix.

The organic solvent includes soybean oil. According to certain embodiments, the organic solvent includes plant-derived oils, saturated fatty acids, unsaturated fatty acids, and mixtures and blends thereof. In further embodiments, the organic solvent includes castor oil, cottonseed oil, medium-chain triglyceride, glyceryl monocaprylocaprate, oleic acid, or linoleic acid, or mixtures or blends thereof.

According to certain embodiments, the organogel composition is adherent to deep tissues.

According to certain embodiments, the organogel composition, at a thickness of approximately 0.5 mm, exhibits an in vitro elution (IVE) profile of the water-soluble antimicrobial or anti-infective agent from the organogel matrix in phosphate buffered saline of between approximately 20% to 100% over a time range of approximately 2 days to 7 days. In additional embodiments, the IVE is approximately 40% or less in the first 24 hours. In further embodiments, the IVE is approximately 80% or greater after 5 days. In still additional embodiments, the IVE is between approximately 10% and 50% per day for days 1 to 3.

The present disclosure, in certain aspects, further describes a method of delivering an active agent to a surgical site including the steps of:
perioperatively compounding solid particles of an active agent within a biocompatible organogel matrix so as to form an organogel drug depot configured for controlled release; and
intraoperatively delivering the organogel drug depot to the surgical site;
where the organogel matrix includes an organogelator and biocompatible organic solvent, and, where the organogel drug depot is in a solid or semisolid state during the step of intraoperative delivery.

According to certain embodiments, the surgical site can include one or more implantable medical devices, such as, for example, an implantable orthopedic device.

According to additional aspects of the present disclosure, a method of preparing a local drug depot having an active agent for delivery to a surgical site includes:
perioperatively compounding solid particles of an active agent within a biocompatible organogel matrix to form an organogel drug depot configured for controlled release;
where the organogel matrix comprises an organogelator and a biocompatible organic solvent, and where the organogel drug depot is in a solid or semisolid state prior to a delivery of the organogel drug depot.

According to certain embodiments, the surgical site can include one or more implantable medical devices, such as, for example, an implantable orthopedic device.

According to certain embodiments, compounding can include heating the organogel matrix to melt the matrix and incorporating the solid particles into the melted matrix. The method can further include, after incorporating the solid particles, cooling the melted matrix to form the organogel drug depot, where the drug depot is in a solid or semisolid state. In some embodiments, cooling the melted matrix occurs within about 10 minutes or less, for example, 5 minutes or less. In alternative embodiments, compounding can include a physical mixing (e.g., mechanical mixing) between the organogel matrix in the solid or semisolid state and the active agent solid particles to form the organogel drug depot, where the drug depot can be in a solid or semisolid state. In still further embodiments, compounding can include a combination of heating and physical or mechanical mixing.

According to certain embodiments, the organogel matrix has a solubility in water of less than 1g/L.

The organogelator includes a mixture of cholesterol and at least one of hydrogenated castor oil or a mono- di- glyceride blend. In certain embodiments, the organogelator includes one or more fatty acids or salts or esters of fatty acids, such as, for example, stearic acid, sodium stearate, oleic acid, sodium oleate, 12-hydroxystearic acid, glyceryl monostearate, glyceryl distearate, or sorbitan monostearate, as well as mixtures thereof. In additional embodiments, the organogelator includes biocompatible molecules such as phospholipids, or hydrogenated phospholipids. In further additional embodiments, the organogelator further includes mono- or di- glycerides having a chain length greater than c10 (an aliphatic carbon molecule having 10 carbon atoms or greater), hydrogenated oils, sodium salts of fatty acids, and combinations thereof.

According to certain embodiments, the biocompatible organic solvent has a melting point below 20 °C, for example the solvent melting point can be below 0°C, or below - 10°C, down to and including -51°C. As such it can be said that suitable biocompatible organic solvents can have a solvent melting point range in some embodiments of between 20°C and -51 °C.

The biocompatible organic solvent includes soybean oil. In certain embodiments, the biocompatible organic solvent can include a biocompatible oil derived from a plant or animal, or synthetic derivatives thereof. In further embodiments, the biocompatible oil includes one or more fatty acids. In still further embodiments, the one or more fatty acids can include unsaturated fatty acids, saturated fatty acids, or a combination or mixture thereof. In some embodiments, the one or more fatty acids can include free fatty acids, or can include fatty acids in the form of triglycerides, or a combination or mixture thereof. In some embodiments, the one or more fatty acids can include free fatty acids, or can include fatty acids in the form of monoglycerides, diglycerides, triglycerides, or a combination or mixture thereof. Exemplary biocompatible organic solvents can include medium chain triglycerides, triolein, glyceryl monocaprylocaprate, ethyl oleate, DL-alpha-tocopherol acetate, linoleic acid, and mixtures thereof. According to certain embodiments, the organic solvent includes plant-derived oils, saturated fatty acids, unsaturated fatty acids, and mixtures and blends thereof. In further embodiments, the organic solvent includes castor oil, cottonseed oil, medium-chain triglyceride, glyceryl monocaprylocaprate, oleic acid, or linoleic acid, or mixtures or blends thereof.

According to certain embodiments, the weight ratio of the organogelator and the biocompatible organic solvent of the organogel matrix is in the range of about 5:95 to about 60:40, such as, for example from about 20:80 to about 50:50. According to certain embodiments, the organogelator comprises less than 50% by weight of the organogel matrix.

The organogel composition comprises solid particles of a water-soluble antimicrobial or anti-infective agent, which may be an antibiotic agent. According to certain embodiments, the active agent is soluble, freely soluble, or very soluble in water, as defined by the United States Pharmacopeia (USP) (i.e., a ratio of water to active agent of about 30:1 or less). In alternative embodiments, the active agent is sparingly soluble, slightly soluble, very slightly soluble, or insoluble in water, as defined by the USP (i.e., a ratio of water to active agent of about 30:1 or more).

According to certain embodiments, the solid particles of the active agent are disposed in within the organic solvent of the organogel matrix. In still further embodiments, the solid particles can have a median D(50) particle size (by volume distribution) in the range of about 1 µm to about 1 mm (1000 microns), such as, for example, in the range of about 1 µm to about 10 µm, or 10 µm to about 50 µm.

According to certain embodiments, the organogel matrix can further include one or more excipients. According to further embodiments, the one or more excipients includes biocompatible surfactants or biocompatible hydrophilic small molecules. In certain embodiments, the one or more excipients can include Poly(ethylene glycol) (PEG), Pluronic F127, Tween 80, Tween 60, Tween 20, or a mixture of any combination thereof.

According to certain embodiments, the organogel matrix is configured to adhere to a metal surface in an aqueous environment. This would include, for example, conditions simulating an *in vivo* aqueous environment. According to certain further embodiments, the organogel matrix is configured to adhere to deep tissue.

According to certain embodiments, the surgical site is an implant site including one or more implantable medical devices, for instance, an implantable orthopedic device. In certain embodiments, an implantable medical device includes a metal surface, and the organogel matrix is configured to adhere to the metal surface *in vivo.* In certain embodiments, the organogel drug depot is intraoperatively delivered to the surgical site via percutaneous syringe injection, such as, for example, through incisions for screw placement in a percutaneous plating procedure. In additional embodiments, the surgical site (with or without an implantable medical device) is operatively opened and the drug depot is intraoperatively delivered to soft or hard tissue at the surgical site, and in procedures involving an implantable medical device at the surgical site, can be delivered adjacent to, or directly onto an outer surface of, an implantable medical device, such as, for example, a metal surface or an orthopedic implant. Typically, orthopedic implants include metal, polymer, or ceramic outer surfaces. In certain additional embodiments, the organogel drug depot is intraoperatively applied onto the implantable device outside the surgical site and then intraoperatively delivered to the surgical site with the implantable medical device.

According to the present disclosure, there is also described a system for preparing an organogel drug depot for local delivery to a surgical site. The system includes an organogel matrix including an organogelator and a biocompatible organic solvent, solid particles of an active agent, a container including at least one wall having an outer surface, where the container defines a volume capable of containing the organogel matrix and active agent solid particles, and a heating component configured to contact the outer surface and supply an amount of heat to the container.

According to certain embodiments, the surgical site is an implant site including one or more implantable medical devices, for instance, an implantable orthopedic device.

In certain embodiments of the system, the container is a syringe. In certain embodiments of the system, the syringe contains a mixing element. In alternative embodiments, the container is a vial.

In still further embodiments, the system can include multiple containers, such that the container is a first container, and an additional container is a second container. In some embodiments, the first container has a first opening and the second container has a second opening, and the first opening is adapted to connect to the second opening.

In additional embodiments, the heating component defines an inner wall. Additionally, the inner wall can include, in some embodiments, at least one heating element, and further that the inner wall is configured to contact the outer surface of the container such that the at least one heating element supplies heat to the organogel matrix.

In certain embodiments, the inner wall defines a substantially cylindrical shape along its length. In still further embodiments, the inner wall defines a first cross-sectional diameter at a first region and a second cross-sectional diameter at a second region, and the first cross-sectional diameter can be greater than the second cross-sectional diameter.

In certain embodiments, the heating element is configured to provide one or more heating profiles along the inner wall, such that the heating component includes at least a first heating profile and a second heating profile.

According to still further embodiments of the present disclosure, methods of delivering an active agent to a non-sterile open wound site are described, including the steps of:
compounding solid particles of an active agent within a biocompatible organogel matrix to form an organogel drug depot; and,
delivering the organogel drug depot to a non-sterile open wound site, where at the time of delivery the open wound site includes soft tissue, hard tissue, or both, that are exposed to a non-sterile environment;
wherein the step of compounding and delivering are performed contemporaneously; and,
wherein the organogel is in a solid or semisolid state during the step of delivering.

According to additional aspects of the present disclosure, there is a method of preparing a local drug depot in a non-sterile environment for delivery of an active agent to a non-sterile open wound site including:
compounding solid particles of an active agent within a biocompatible organogel matrix to form an organogel drug depot;
wherein the step of compounding is performed contemporaneous to a delivery; and,
wherein the organogel is in a solid or semisolid state during compounding.

According to certain embodiments, contemporaneous compounding and delivery are within two hours or less of each other, for example within 1.5 hours, with 1.0 hours, or within 0.5 hours.

According to certain embodiments, the compounding comprises heating the organogel matrix to melt the matrix and incorporating the solid particles into the melted matrix. In further embodiments, the method further comprises, after incorporating the solid particles, cooling the melted matrix to form the organogel drug depot. In certain additional embodiments, cooling the melted matrix is about 10 minutes or less.

According to certain embodiments, compounding comprises a physical mixing between the organogel matrix in solid or semisolid state and the solid particles.

According to certain embodiments, the organogel matrix has a solubility in water of less than 1g/L.

In certain embodiments, the organogel matrix is configured to adhere to the soft tissue, hard tissue, or both, in a substantially aqueous environment

In preferred embodiments, the active agent is selected from Cephalosporins, Aminoglycosides, Glycopeptides, Fluoroquinolones, Lipopeptides, Carbapenems, Rifamycins, as well as Antifungals, and combinations thereof. Suitable exemplary active agents can include cefazolin, cefuroxime, amikacin, gentamicin, tobramycin, vancomycin, ciprofloxacin, moxifloxacin, daptomycin, meropenem, ertapenem, rifampin, amphotericin-B, and fluconazole.

In additional embodiments, the active agent is soluble, freely soluble, or very soluble in water. According to alternative embodiments, the active agent is sparingly soluble, slightly soluble, very slightly soluble, or insoluble in water. In still further embodiments, the active agent solid particles have a D(50) median particle size distribution in the range of 1 µm to about 1 mm.

According to certain embodiments, the organogel matrix further comprises one or more excipients. In certain embodiments, the one or more excipients includes biocompatible surfactants or biocompatible hydrophilic small molecules, or a combination thereof. In still further embodiments, the one or more excipients includes Poly(ethylene glycol) (PEG), Pluronic F127, Tween 80, or a mixture of any combination thereof.

According to certain embodiments, the contemporaneous compounding and delivering are within 1.5 hours or less of each other. In still further embodiments, the contemporaneous compounding and delivering are within 1.0 hours or less, and can be within 0.5 hours or less.

### BRIEF DESCRIPTION OF THE DRAWINGS

The drawings illustrate generally, by way of example, but not by way of limitation, various embodiments discussed in the present disclosure. The foregoing summary, as well as the following detailed description of preferred embodiments of the application, will be better understood when read in conjunction with the appended drawings:
Fig. 1 is a front perspective view of a heating component according to one embodiment having a C-clip configuration;
Fig. 2A is a front perspective view of heating component according to another embodiment including an elastomeric step-tapered configuration;
Fig. 2B is a cross-section side view of the heating component of Fig. 2A;
Fig. 3 is a perspective view of another embodiment of a heating component having a hinge-shaped configuration;
Fig. 4A is a perspective view of heating device including a cradle shaped base unit with two connected syringes in an upright configuration and a drug-loading funnel;
Fig. 4B is a perspective view of the cradle-shaped heating device of Fig. 6A in a different configuration, including the heating component of Fig. 3 disposed in the base unit and retaining one of the syringes;
Fig. 4C is a cross-sectional view of the cradle-shaped base unit of Fig. 4A;
Fig. 5 is a front view of a heating device for use with a vial including a luer lock adapter cap;
Fig 6A is a front perspective view of a heating device for use with a syringe and a stand including a heating component configured to attachably couple to a base unit with a drug-loading funnel;
Fig 6B is a front perspective view of the heating device of 6A assembled for heating and melt-mixing;
Fig. 7 is a perspective view of a heating device including a heating component configured to attachably couple to a base unit;
Fig. 8A is a photograph of an organogel matrix that has been applied and adhered to the bottom of a metal weigh boat filled with phosphate buffered saline (PBS);
Fig. 8B is a photograph of three organogel matrix formulations that are adhered to the bottom of a metal weigh boat after exposure to a spray of deionized water;
Fig. 9A is a photograph showing the application of an organogel matrix including toluidine blue O dye applied onto a metal bone plate and surrounding tissue of a chicken thigh;
Fig. 9B is a photograph showing the applied organogel matrix of Fig. 9A after irrigation and manual rubbing of the bone plate with the skin closed over the plate;
Fig. 10A is a photograph showing the percutaneous injection of an organogel matrix including toluidine blue O dye applied through a skin incision of a chicken thigh;
Fig. 10B is a photograph showing distribution of the organogel matrix to the exposed muscle and fascia of the chicken thigh of Fig. 10A after percutaneous injection;
Fig. 10C is a cross section of muscle tissue recovered after a subcutaneous injection of organogel;
Fig. 10D is a photograph of organogel matrix containing toluidine blue O dye on chicken muscle and hypodermis tissue;
Fig. 11 is a photograph showing reconstitution of a semisolid organogel matrix from a melt state over the course of 5 minutes;
Fig. 12A is a differential scanning calorimeter graph showing temperature and heat values for an organogel matrix;
Fig. 12B is a differential scanning calorimeter graph showing temperature and heat values for the organogel matrix formulation of Fig. 12A including the addition of excipients;
Fig 13 is a photograph of a battery-powered heating device melting 6 grams of organogel matrix in approximately 2 minutes;
Fig. 14A is a graph showing the 14-day cumulative release profiles of gentamicin sulfate from three organogel drug depot formulations mixed at room temperature;
Fig. 14B is a graph showing the 14-day cumulative release profiles of gentamicin sulfate from three melt-mixed organogel drug depot formulations;
Fig. 14C is a graph comparing the release profiles of the three organogel drug depot formulations of Fig. 14B against the release profiles from two published hydrogel systems;
Fig. 15 is a graph showing the 7-day cumulative release profiles of four melt-mixed organogel drug depots formulations;
Fig. 16 is a graph of the log reduction in colony forming units (CFU) of a 3-day staphylococcus aureus biofilm grown on an orthopedic implant from systemic levels of gentamicin versus gentamicin delivered from an organogel;
Fig. 17 is a graph of the 3-day cumulative release of moxifloxacin hydrochloride from high melting formulations from a 3mm thick organogel matrix layer;
Fig. 18 is a graph of the 3-day cumulative release profiles of moxifloxacin hydrochloride from four semisolid mixed organogel drug depot formulations with varying components from a 0.5mm thick organogel matrix layer;
Fig. 19 is a graph of the 3-day cumulative release of moxifloxacin hydrochloride from formulations with a varying mass percentage of glyceryl monocaprylocaprate
Fig. 20 is a graph of the 3-5-day cumulative release of moxifloxacin hydrochloride from formulations with varying mass percentage of oleic acid; and,
Fig. 21 is a graph of the 7-day cumulative release profiles of moxifloxacin hydrochloride from exemplary gamma-irradiated formulations from a 0.5mm thick layer;

### DETAILED DESCRIPTION

In this document, the terms "a" or "an" are used to include one or more than one and the term "or" is used to refer to a nonexclusive "or" unless otherwise indicated. In addition, it is to be understood that the phraseology or terminology employed herein, and not otherwise defined, is for the purpose of description only and not of limitation. When a range of values is expressed, another embodiment includes from the one particular value and/or to the other particular value. Similarly, when values are expressed as approximations, by use of the antecedent "about," it will be understood that the particular value forms another embodiment. All ranges are inclusive and combinable. Further, reference to values stated in ranges includes each and every value within that range. It is also to be appreciated that certain features of the invention, which, for clarity, are described herein in the context of separate embodiments, may also be provided in combination in a single embodiment. Conversely, various features of the invention that are, for brevity, described in the context of a single embodiment, may also be provided separately or in any subcombination.

Descriptive terms related to the solubility of a given solute in a given solvent are made with reference to the use of those terms as understood and used by the United States Pharmacopeia (USP) as follows:
"Very Soluble" as used herein means less than one part of solvent is required for one part of solute. "Freely Soluble" as used herein means that from about 1 to about 10 parts of solvent is required for one part of solute. "Soluble" as used herein means that from about 10 to about 30 parts of solvent is required for one part of solute. "Sparingly Soluble" as used herein means that from about 30 to about 100 parts of solvent is required for one part of solute. "Slightly Soluble" as used herein means that from about 100 to about 1,000 parts of solvent is required for one part of solute. "Very Slightly Soluble" as used herein means that from about 1,000 to about 10,000 parts of solvent is required for one part of solute. "Practically Insoluble" or "Insoluble" as used herein means that greater than or equal to about 10,000 parts of solvent is required for one part of solute.

As used herein "semisolid" when used in describing properties of the organogel, means that the organogel matrix, or the organogel drug depot, does not flow without extrinsic application of force, yet the material will flow upon application of force, such as, for example, upon dispensing from a syringe or manual spreading across tissue within a surgical site. This definition includes, but is not limited to, Bingham plastics.

As used herein "melt" is the state change of the solid or semisolid organogel matrix or organogel drug depot to a liquid state.

As used herein, "organogelator" is a solid or semisolid organic compound defined by its monomeric subunit, which, when placed in contact with a biocompatible organic solvent, such as an oil, forms networks that act to stabilize the organic solvent, forming an organogel. In certain embodiments, the network is a three-dimensional fibrillar network. In certain embodiments, the organogelator can be characterized as lipid crystals.

As used herein, "organogel matrix" is a gel composed of at least an organogelator and a biocompatible organic solvent, such as an oil. The organogelator according to the present disclosure can further include one or more excipients. While it is commonly understood that an organogel matrix will typically constitute a majority percentage by weight of the biocompatible organic solvent, for the purpose of this disclosure, the organogel matrix described herein can, in some embodiments, include equal amounts of each component, and in further embodiments, the organogelator can be a majority constituent by weight.

As used herein, "intraoperative" means the time period during a surgical procedure.

As used herein, "perioperative" means the time frame during the course of a surgical procedure (i.e., intraoperative), as well as a reasonable time period prior to the surgical procedure. For the purposes of this disclosure, a reasonable time period can be considered within six to eight hours of the surgical procedure.

As used herein "contemporaneous" means within 2 hours or less, such that the delivery of the organogel drug depot to the soft or hard tissue or both, will be within any time period within 2 hours or less from the start of the preparation of the organogel drug depot, for example, 1.5 hours, 1.0 hour, 45 minutes, 30 minutes, 20 minutes, or 10 minutes, or any range or combination of ranges within 2 hours or less.

As used herein "non-sterile" means an environment, location, or surface that is not free from viruses, bacteria, foreign bodies, or any other potentially infection causing components.

As used herein "open wound" means a traumatic injury where the skin is torn, cut, or punctured such that the dermis is damaged, and the underlying fascia, muscle, bone, or other internal organs (for purposes herein referred to as "deep tissue") are exposed to the external environment. Such open wounds can be the result of lacerations, abrasions, avulsions, punctures, or penetrations to the skin and can have a likelihood of contamination.

The present disclosure describes an organogel composition for application to an open-wound site in a non-sterile environment, particularly an open-wound exposing deep tissue, including:
solid particles of a water-soluble antimicrobial or anti-infective agent; and,
an organogel matrix, wherein the solid particles are dispersed within the organogel matrix,
where the organogel matrix includes a biocompatible organogelator and a biocompatible organic solvent, the organogel matrix having a melting point peak as measured by differential scanning calorimetry in the range of about 47° C to about 102° C,
wherein the biocompatible organogelator includes a mixture of cholesterol and at least one of hydrogenated castor oil or a mono- di- glyceride blend, and
wherein the biocompatible organic solvent includes soybean oil.

In further embodiments, the present disclosure describes an organogel matrix containing solid particles of of a water-soluble antimicrobial or anti-infective agent for use as a local drug depot at a surgical site. The disclosed organogel drug depot provides the advantage of a controlled release matrix that is biocompatible, hydrophobic, tissue-adherent, implant adherent, and migration resistant, can be injectable, or applied manually, and does not inhibit healing at the surgical site. The disclosed delivery process of the present disclosure has the further advantage of permitting the medical professional to select an active agent and release rate based upon an individual patient's specific needs and risk factors in contrast to pre-coated, or other types of pre-loaded, or fixed dose medical implants.

An additional advantage of the disclosed organogel drug depot and delivery process is that it permits the contemporaneous preparation and delivery to a non-sterile open wound site, such as an acute traumatic injury site (e.g., combat injury or machine accident) with desired adherence to the tissue at the wound site to achieve the necessary therapeutic effect, such as for example infection prevention or pain relief.

The organogel matrix has the advantages of low-temperature melting, tunable-release, and a variety of strategies for room temperature or melt reconstitution of active agent particles (e.g., Active Pharmaceutical Ingredient (API) powders) that enables the medical professionals to formulate an antibacterial, anesthetic, or other drug delivery depot perioperatively, and particularly intraoperatively. Moreover, the organogel matrices allow for application and retention to both hard and soft tissue surfaces, as well as metal surfaces in aqueous environments such as *in vivo* conditions. This permits implantable medical devices, such as implantable orthopedic devices to be coated with the organogel drug depot after completion of internal fixation and prior or subsequent to final irrigation before closure; or, alternatively to be coated with the organogel drug depot prior to implantation of the medical device, such that the delivery of the organogel drug depot and the implantable medical device to the surgical site occurs simultaneously.

For example, in certain embodiments, the organogel drug depot may be prepared within 15 minutes and is stable enough to allow for preparation up to at least 6-8 hours ahead of delivery to the surgical site, or the non-sterile traumatic injury site, such as one including expose deep tissue. This allows for intraoperative or perioperative preparation of the organogel drug depot such that all available patient data can be included in the selection of the drug molecule and delivery duration at or near the time of delivery. It should be appreciated that, in certain other embodiments, the organogel matrix could be prepared in a time period prior to a perioperative time period, such as for example, a manufacturer of an organogel matrix could prepare the composition at an offsite location and ship the composition to the surgical location, which at that point the perioperative compounding of the organogel matrix with the solid particles of an active agent can then occur.

The organogel drug depot of the present disclosure can additionally provide sufficient duration of active agent delivery clinically-relevant to local prevention of bacterial colonization or pain relief; typically within the range of about 1-14 days, and have sufficient dose strength to protect both the tissue surrounding the surgical site, and where applicable any implantable medical devices at the surgical site, such as in the case where antimicrobials, antibiotics, or local anesthetics are the desired active agents of interest. For example, in certain embodiments, the organogel drug depot can be configured for acute dosing, such as for example, less than 6 hours, or less than 12 hours, or less than 1 day to about 1-3 days. In certain other embodiments, the organogel drug depot can be configured for an intermediate dosing period, such as for example, in the range of 4-7 days. In additional embodiments, the organogel drug depot can be configured for a longer-term dosing period, such as for example, 7-14 days. In still further additional embodiments, the organogel drug depot can be configured for an extended-release dosing period of up to 3-4 weeks. It should be appreciated that in embodiments where multiple active agents are utilized in the organogel drug depot, the organogel drug depot can be configured to have multiple dosing profiles (e.g., acute and long term) based upon the release profile of the selected active agents compounded within the organogel drug depot. Additionally, the organogel drug depot of the present disclosure has a sufficiently low bulk mass to allow for standard surgical soft tissue closure techniques at the surgical site as compared to use of antibiotic loaded cements as previously described. Furthermore, the organogel matrix can permit controlled release of multiple active agents having different properties such as molecular weight, log P values, etc., that would typically result in different release profiles in vivo.

In yet further embodiments of the present disclosure, the organogel drug depot has a lower limit to its viscosity range that is sufficiently high such that without application of extrinsic force the organogel drug depot exhibits substantially no flow. Furthermore, the organogel drug depot has an upper limit to its viscosity range that is sufficiently low such that application of mechanical force (e.g., a hand or surgical tool or device) to the organogel drug depot permits the even spreading or distribution (i.e., shearing) of the organogel drug depot to the necessary locations in and around the surgical site, such as the soft or hard tissues, or any implantable medical devices at the surgical site. In certain embodiments, the application of the organogel matrix to deep tissue is such that it is adherent to deep tissue even in a substantially aqueous environment, e.g., in the presence of blood and/or irrigation.

According to the present disclosure, a method of delivering an active agent to a surgical site is described including the steps of:
perioperatively compounding solid particles of an active agent within a biocompatible organogel matrix so as to form an organogel drug depot configured for controlled release; and
intraoperatively delivering the organogel drug depot to the surgical site;
where the organogel matrix includes an organogelator and a biocompatible organic solvent; and,
where the organogel drug depot is in a solid or semisolid state during the step of intraoperative delivery.

The organogel matrix includes an organogelator and a biocompatible organic solvent as defined in the appended claims. An organogelator can be a low molecular-mass organic gelators (LMOGs). LMOGs are characterized by their ability to form self-assembled gel networks, such as for example, fibrillar networks. The ability to self-assemble can occur from the formation of non-covalent interactions between the individual monomeric sub-units. An organogelator can include a lipid crystal network of a higher-melting component that can be dissolved or melted in the biocompatible organic solvent at an elevated temperature and where a lipid crystal network forms upon cooling. According to certain embodiments, organogelators can include fatty acids and derivatives thereof. For example, considering the fatty acid steric acid as an example, embodiments would include stearic acid (fatty acid), sodium stearate (fatty acid salt), and sorbitan monostearate (fatty acid ester). Organogelators can also include n-alkanes. In additional embodiments, the organogelator can include biocompatible molecules such as phospholipids. In further embodiments, the organogelator can include saturated triglycerides or hydrogenated oils such as tristearin or hydrogenated castor oil.

According to certain embodiments, the organogelator includes one or more fatty acids or salts or esters of fatty acids, such as, for example, stearic acid, sodium stearate, oleic acid, sodium oleate, 12-hydroxystearic acid, glyceryl monostearate, glyceryl distearate, or sorbitan monostearate, as well as mixtures thereof. In additional embodiments, the organogelator includes biocompatible molecules such as phospholipids, or hydrogenated phospholipids. In further additional embodiments, the organogelator further includes mono- or di- glycerides having a chain length greater than c10 (an aliphatic carbon molecule having 10 carbon atoms or greater), hydrogenated oils, sodium salts of fatty acids, and combinations thereof.

In certain embodiments for use in non-sterile environments, it can be preferable that the melting point is at least about 49 °C, for example in the range of about 49 °C to about 71 °C, or about 49 °C to about 100 °C, as well as any combination or subcombination of endpoints disclosed herein.

The biocompatible organic solvent includes soybean oil. According to certain embodiments, the biocompatible organic solvent is an organic solvent approved for use in humans by the U.S. Food and Drug Administration. In certain embodiments, the biocompatible organic solvent includes a plant or animal-based oil or a synthetic derivative thereof. Examples can include cottonseed oil, or castor oil. In certain embodiments, the oil includes one or more fatty acids or fatty acid esters. In still further embodiments, the one or more fatty acids can include unsaturated fatty acids, saturated fatty acids, or a combination or mixture thereof. In some embodiments, the one or more fatty acids can include free fatty acids, or can include fatty acids in the form of monoglycerides, diglycerides, triglycerides, or a combination or mixture thereof. Exemplary biocompatible organic solvents can include linoleic acid, medium chain triglycerides, triolein, glyceryl monocaprylocaprate, ethyl oleate, DL-alpha-tocopherol acetate, and mixtures thereof.

According to further embodiments, the biocompatible organic solvent can include a biocompatible oil derived from a plant or animal, or synthetic derivatives thereof. In still further embodiments, the biocompatible oil includes one or more fatty acids. In still further embodiments, the one or more fatty acids can include unsaturated fatty acids, saturated fatty acids, or a combination or mixture thereof. In some embodiments, the one or more fatty acids can include free fatty acids, or can include fatty acids in the form of triglycerides, or a combination or mixture thereof. In some embodiments, the one or more fatty acids can include free fatty acids, or can include fatty acids in the form of monoglycerides, diglycerides, triglycerides, or a combination or mixture thereof. Exemplary biocompatible organic solvents can include medium chain triglycerides, triolein, glyceryl monocaprylocaprate, ethyl oleate, DL-alpha-tocopherol acetate, linoleic acid, and mixtures thereof. According to certain embodiments, the organic solvent includes plant-derived oils, saturated fatty acids, unsaturated fatty acids, and mixtures and blends thereof. In further embodiments, the organic solvent includes castor oil, cottonseed oil, medium-chain triglyceride, glyceryl monocaprylocaprate, oleic acid, or linoleic acid, or mixtures or blends thereof.

In still further embodiments, the oil has a melting point from below 20 °C to below -51 °C. In other embodiments the oil has a melting point from below 0 °C to below -51 °C, and preferably from below -10 °C to below -51 °C.

The organogel composition comprises solid particles of a water-soluble antimicrobial or anti-infective agent. In preferred embodiments, the active agent is selected from Cephalosporins, Aminoglycosides, Glycopeptides, Fluoroquinolones, Lipopeptides, Carbapenems, Rifamycins, as well as Antifungals, and combinations thereof. Suitable exemplary active agents can include moxifloxacin, cefazolin, cefuroxime, amikacin, gentamicin, tobramycin, vancomycin, ciprofloxacin, moxifloxacin, daptomycin, meropenem, ertapenem, rifampin, amphotericin-B, and fluconazole.

According to certain embodiments, the active agent is soluble, freely soluble, or very soluble in water, as defined by the United States Pharmacopeia (USP). In alternative embodiments, the active agent is sparingly soluble, slightly soluble, very slightly soluble, or insoluble in water, as defined by the USP.

According to certain embodiments, the solid particles of the active agent are disposed within the organic solvent component of the organogel matrix. In still further embodiments, the solid particles can have a D(50) median particle size (by volume distribution) in the range of about 1-1000 µm, such as, for example, in the range of about 1 µm to about 10 µm, about 1 µm to about 5 µm, about 5 µm to about 10 µm, about 10 µm to about 20 µm, about 10 µm to about 50 µm, about 1 µm to about 50 µm, about 50 µm to about 100, about 1 µm to about 100 µm, about 100 µm to about 500 µm, or about 100 µm to about 1000 µm.

In certain embodiments, the organogel drug depot has an active agent content in the range of about 1% to 30% by weight. In certain embodiments, the active agent content can be in the range of 1% to 5%, 1% to 10%, 5% to 10%, 10% to 20%, 5% to 20%, 10% to 30%, 20% to 30%, about 10%, about 20%, or about 25%, for example, or any combination of ranges listed above.

According to certain embodiments, the organogel matrix is very slightly soluble or insoluble in water, such that, for example, the organogel matrix has a solubility in water of less than 1g/L. According to further embodiments, the organogel matrix can have a weight ratio of organogelator to biocompatible organic solvent in the range of about 5:95 to about 70:30. In still further embodiments, the weight ratio can be in the range of about 30:70 to about 50:50. For example the weight ratio can be 10:90, 25:75, 30:70, 40:60, 45:55, 50:50, 55:45, 60:40, or 70:30.

According to certain embodiments, the organogel composition, at a thickness of approximately 0.5 mm, exhibits an in vitro elution (IVE) profile of the water-soluble antimicrobial or anti-infective agent from the organogel matrix in phosphate buffered saline of between approximately 20% to 100% over a time range of approximately 2 days to 7 days. In additional embodiments, the IVE is approximately 40% or less in the first 24 hours. In further embodiments, the IVE is approximately 80% or greater after 5 days. In still additional embodiments, the IVE is between approximately 10% and 50% per day for days 1 to 3.

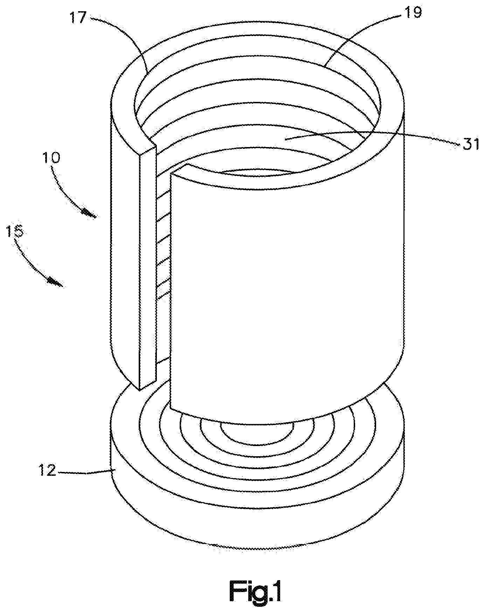

According to the present disclosure, and with reference to Figs. 1-2, in certain embodiments, compounding can include heating the organogel matrix to melt the matrix and incorporating (e.g., suspending) the solid particles into the melted matrix. The method can further include, after incorporating the solid particles, cooling the melted matrix to form the organogel drug depot, where the drug depot is in a solid or semisolid state. In further embodiments, perioperative compounding is intraoperative compounding. In some embodiments, cooling the melted matrix occurs within about 10 minutes or less, for example, 5 minutes or less. In alternative embodiments, compounding can include a physical mixing between the organogel matrix in solid or the semisolid state and the solid particles to form the organogel drug depot, where the drug depot is in a solid or semisolid state. In still further embodiments, compounding can include a combination of heating and physical mixing.

According to certain embodiments, the organogel matrix can further include one or more excipients. In certain embodiments, the one or more excipients includes biocompatible surfactants or biocompatible hydrophilic small molecules, or a combination thereof. In still further embodiments, the biocompatible hydrophilic small molecules can increase the water-solubility of the matrix. In further embodiments, the small molecule has a weight average molecular weight of about 20,000 Daltons (20kD) or less. In certain embodiments, the one or more excipients can include PEG₁₀ₖ, Pluronic F127, Tween 80, Tween 60, Tween 20, or a mixture of any combination thereof.

According to certain embodiments, the organogel drug depot is intraoperatively delivered to the surgical site via percutaneous syringe injection through a cannula. In additional embodiments, the surgical site (with or without an implantable medical device) is operatively open and the drug depot is intraoperatively delivered onto soft or hard tissue at the surgical site. In procedures including one or more implantable medical devices, the intraoperative delivery of the organogel drug depot can additionally include delivery adjacent to, or directly onto, an outer surface of an implantable medical device, such as, for example, a metal surface or an orthopedic implant. In certain additional embodiments, the organogel drug depot is first intraoperatively applied onto the implantable device outside the surgical site and then intraoperatively delivered to the surgical site with the implantable medical device.

According to the present disclosure, there is also described a system for preparing an organogel drug depot for local delivery to a surgical site or as has also been described, for local delivery to a non-sterile open wound site, such as a wound site exposing deep tissue to a non-sterile environment. The system includes an organogel matrix including an organogelator and an oil, solid particles of an active agent, a container including at least one wall having an outer surface, where the container defines a volume capable of containing the organogel and active agent solid particles, and a heating element configured to contact the outer surface and supply an amount of heat to the container.

In certain embodiments of the system, the container is a syringe. In alternative embodiments, the container is a vial. In certain instances, the container can be formed specifically to compliment the shape of a heating component. In certain other instances, the vial can be the original drug manufacture vial.

In still further embodiments, the system can include multiple containers, such that the container is a first container, and an additional container is a second container. In some embodiments, the first container has a first opening and the second container has a second opening, and the first opening is adapted to connect to the second opening.

With reference to Figs. 1-3, a heating component 10 is disclosed, the heating component 10 defining an inner wall 17. The inner wall 17 can include, in some embodiments, at least one heating element 19, and further that the inner wall 17 is configured to contact the outer surface of the container (not shown) such that the at least one heating element 19 supplies heat to the organogel matrix.

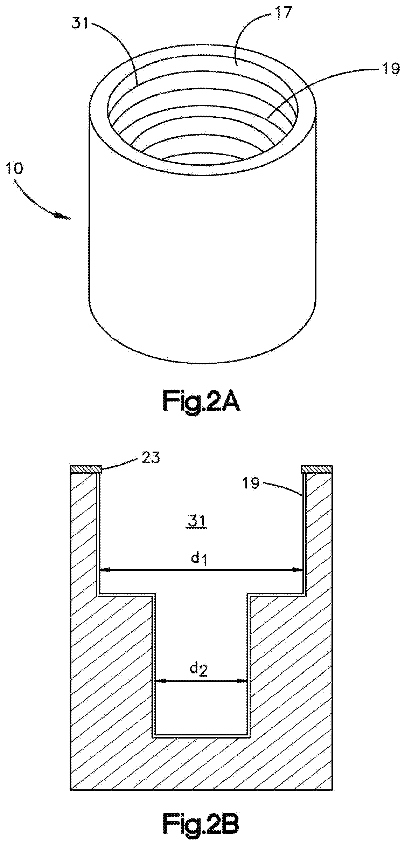

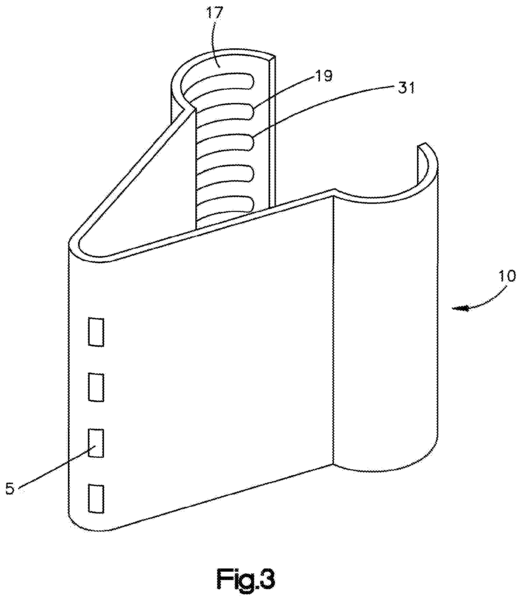

In certain embodiments, such as is shown in Figs. 1 and 3, the inner wall 17 defines a substantially uniform cylindrical shape along the length of the heating component 10. In still further embodiments, such as is shown in Figs. 2A-B, the inner wall 17 can define a nonuniform cross section, such that for example, the inner wall 17 defines a first cross-sectional diameter d₁ at a first region and a second cross-sectional diameter d₂ at a second region, and the first cross-sectional diameter can be greater than the second cross-sectional diameter.

In certain embodiments, the heating element 19 is configured to provide a uniform heating profile substantially along the length of the heating component 10. In other embodiments, the heating element 19 is configured such that it can provide one or more heating profiles along the inner wall 17, such that the heating device 10 includes at least a first heating profile and a second heating profile.

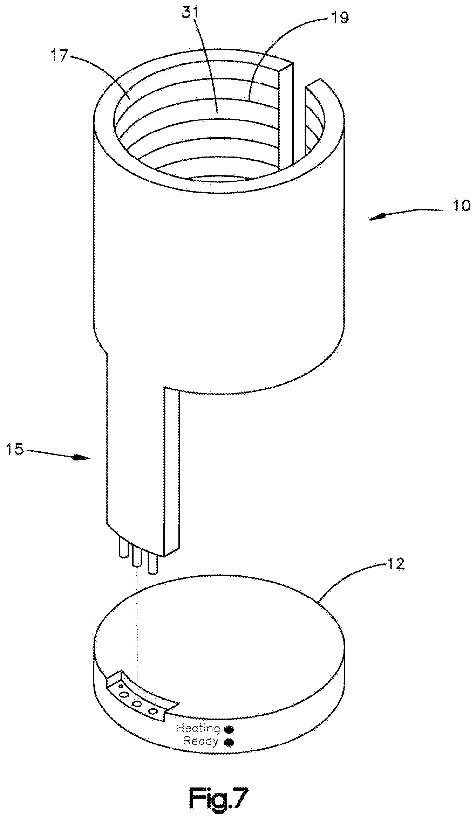

Referring to Fig. 1, a heating device 15 is shown including a heating component 10 configured in the shape of a C-clip, and a base unit 12. According to certain embodiments, and as shown in Fig. 1, heating component 10 and base unit 12 are integrally formed into a monolithic heating device 15. In alternative embodiments, such as shown in Fig. 7, heating component 10 and base unit 12 are configured such that heating component 10 can attachably couple to base unit 12. Base unit 12 can, in certain embodiments, house a power supply and electronics necessary to supply energy to the heating component and to configure one or more heating profiles for the heating component 10. In certain other embodiments, the base unit 12 is optional, such that the heating device 15 consists only of the heating component 10. In these embodiments, the heating component 10 can provide its own power to generate heat. The heating component 10, according to one embodiment defines a substantially cylindrical shaped inner wall 17 along its length that includes one or more heating elements 19 disposed along the length of its surface. The inner wall 17 defines a cavity 31 shaped to accept a container (not shown), such as for example, a syringe or a vial. Because the heating component 10 has a C-clip configuration, which can rely on a snap-fit or friction-fit engagement with the container, it can accommodate containers having a range of cross-sectional diameters.

Referring to Figs. 2A-B, a heating component 10 is shown configured in the shape of a tiered chamber. The heating component 10 further defines an inner wall 17 including one or more heating elements 19 along its length. The inner wall 17 defines a cavity 31 having one or more cross-sectional diameters along its length such that the heating component 10 can include a first cross-sectional diameter d₁ at a first region and a second cross-sectional diameter d₂ at a second region, and wherein the first cross-sectional diameter is greater than the second cross-sectional diameter. The heating component 10 is therefore configured, according to certain embodiments, to accept containers (not shown) having a smaller cross-section diameter in the second region, and accept containers having a greater cross-sectional diameter in the first region. The heating component 10 can further include, in certain embodiments, one or more lips 23 that extend into the cavity region 31 such that the lips are adapted to secure the container, for example, by a friction fit or other mechanical restraint.

Referring to Fig. 3, a heating component 10 is shown configured in the shape of a living hinge (or clamshell hinge). The heating component 10 further defines an inner wall 17 including one or more heating elements 19. The inner wall 17 defines a cavity 31 shaped to accept and secure a container (not shown) through a mechanical friction fit. Because the heating component 10 is configured in the shape of a hinge, it can accommodate containers having a range of cross-sectional diameters.

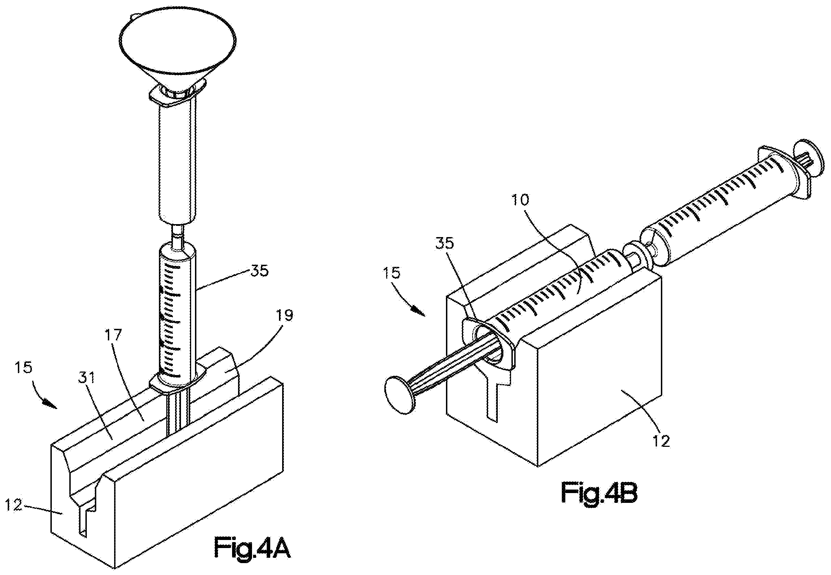

Referring to Figs. 4A-C, a heating device 15 is shown having a base unit 12 configured in the shape of an elongated cradle. According to certain embodiments, as shown in Fig. 4A, the heating device 15 further includes a heating component 10 integrally formed with base unit 12 such that the heating component 10 and base unit 12 form a single integral body. Heating device 15 further defines an inner wall 17 including one or more heating elements 19. The inner wall 17 defines a cavity 31 shaped to accept a container 35. Further, as shown in Fig. 4A, the inner wall 17 of the device body 15 is dimensioned to allow a container 35 (shown here as a syringe) to be secured in an upright position to allow for the container 35 to be filled with either the organogel matrix, the active agent, or both.

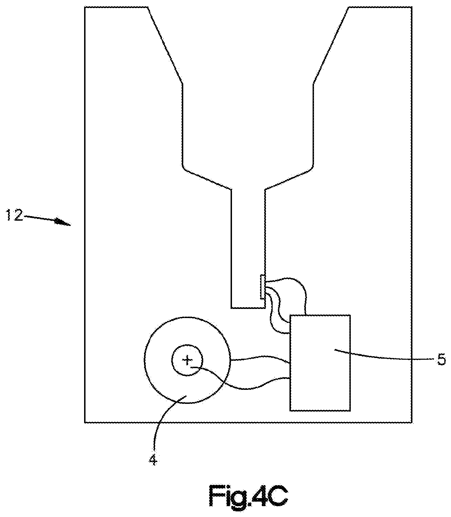

According to certain embodiments, as shown in Fig. 4B, the heating device 15 can include a base unit 12 configured in the shape of a cradle, where the base unit 12 is dimensioned to allow heating component 10 (as shown here, the hinged heating component of Fig. 3) to attachably couple to base unit 12. Additionally, as shown, the inner wall 17 of heating component 10 is dimensioned to allow the container 35 to be positioned within the cavity 31 such that the container 35 is in contact with the heating elements 19 of the inner wall 17 of the heating component 10. Fig. 4C shows one embodiment of the base unit 12 housing a battery 4 and the corresponding electronics 5 utilized to provide energy to the heating component 10 when base 12 and heating component 10 are operatively coupled together.

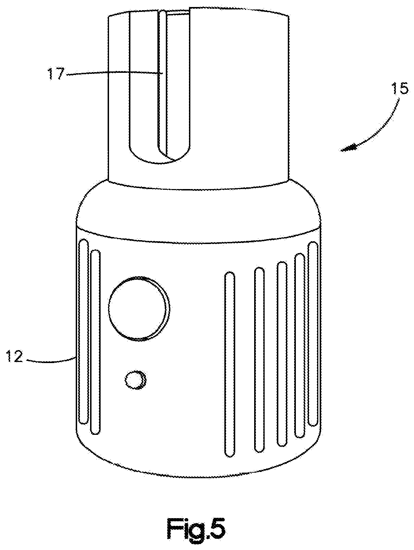

Referring to Fig. 5, a heating device 15 is shown including a heating component (not shown) integrally formed with base 12. Inner wall 17 defines a cavity (not shown) to receive a container (not shown). Additionally, the heating device 15 can include a luer lock adapter cap system to facilitate the connection of a first container, for example, a vial, to a second container, for example, a syringe. It should be appreciated that heating component 10 could be detachably coupled to base 12, such as for example, the heating components shown in Figs. 1-2, being slidably inserted into base 12, in order to accommodate a container having a corresponding shape as desired.

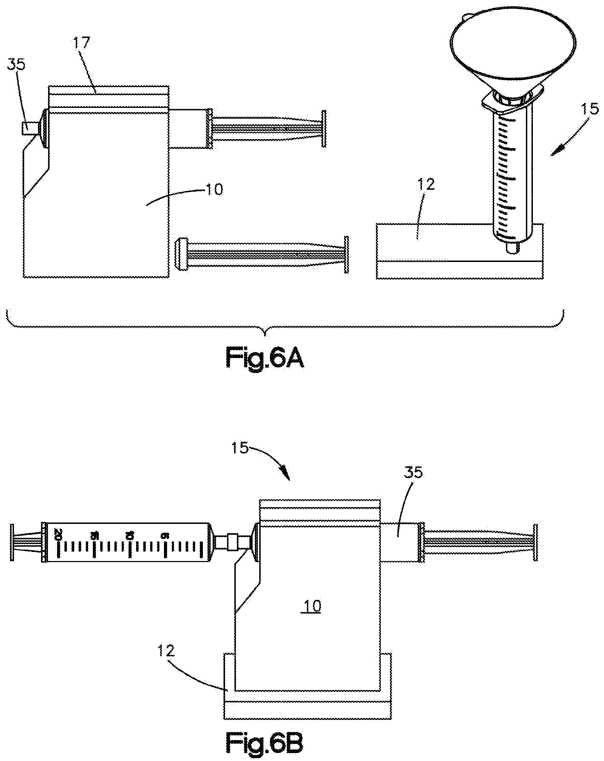

Referring to Figs. 6A-B, a heating device 15 is shown including a heating component 10 and base unit 12. As shown in Fig. 6A, heating component 10 is detached from base 12. Heating component 10 includes an inner wall 17 defining a cavity (which as shown here, is occupied with container 35, shown as a syringe). The container 35 is in contact with heating elements 19 (not shown) disposed along the inner wall. Heating component 10, according to certain embodiments, and as shown here, can be shaped and dimensioned to include batteries 4 (not shown but contained within) to supply power. Base 12, can include in certain embodiments, a stand or mounting aid, for container 35 to assist a user in preparing the organogel compositions. Base 12 can further include the necessary electronics 5 for providing one or more heating profiles to the heating elements 19. As shown in Fig. 6B, base 12 and heating component 10 are connected such that a heating profile can be delivered to container 35 disposed within cavity 31.

Referring to Fig. 7, a heating device 15 is shown having a heating component 10 and base unit 12 that can be attachably coupled. Base unit 12 can include a power supply and the necessary electronics to provide one or more heating profiles to heating component 10. The heating component further defines an inner wall 17 including one or more heating elements 19. The inner wall 17 defines a cavity 31 shaped to accept and secure a container (not shown). The heating device 15 can be configured such that the base unit 12 provide a heating profile to the heating component 10 when they are operatively coupled. Alternatively, the base unit 12 can charge the heating component 10 with sufficient power such that heating component 10 can heat the container if it is detached from base unit 12. In other words, the heating component 10 can be portable and separable from the base unit 12 and still provide heat to the container.

According to the present disclosure, methods of delivering an active agent to a non-sterile open wound site are described including
compounding solid particles of an active agent within a biocompatible organogel matrix to form an organogel drug depot; and,
delivering the organogel drug depot to an open wound site, wherein at the time of delivery the open wound site includes soft tissue, hard tissue, or both, that are exposed to a non-sterile environment;
wherein the step of compounding and the step of delivering are performed contemporaneously; and,
wherein the organogel is in a solid or semisolid state during the step of delivering.

According to other embodiments of the present disclosure, methods of preparing a local drug depot in a non-sterile environment for delivery of an active agent to a non-sterile open wound site comprising:
compounding solid particles of an active agent within a biocompatible organogel matrix to form an organogel drug depot;
wherein the step of compounding is performed contemporaneous to a delivery; and,
wherein the organogel is in a solid or semisolid state during compounding.

According to certain embodiment the contemporaneous compounding and delivering are performed within any time period within 2 hours or less from the start of the preparation of the organogel drug depot, for example, 1.5 hours, 1.0 hour, 45 minutes, 30 minutes, 20 minutes, or 10 minutes, or any range or combination of ranges within 2 hours or less.

According to certain embodiments, the open wound site can include exposed soft tissue, hard tissue, and fascia, as well as other underlying internal organs, the surfaces of which each are suitable for delivery of the organogel drug depot.

It should be appreciated that the previously disclosed components of the organogel drug depot, its properties, apply equally to this method of treatment of preparing and delivering an active agent to a non-sterile open wound site.

As such, according to certain embodiments, contemporaneous compounding and delivery are within two hours or less of each other, for example within 1.5 hours, with 1.0 hours, or within 0.5 hours.

According to certain embodiments, the compounding comprises heating the organogel matrix to melt the matrix and incorporating the solid particles into the melted matrix. In further embodiments, the method further comprises, after incorporating the solid particles, cooling the melted matrix to form the organogel drug depot. In certain additional embodiments, cooling the melted matrix is in about 10 minutes or less.

According to certain embodiments, compounding comprises a physical mixing between the organogel matrix in solid or semisolid state and the solid particles. In certain embodiments, physical mixing can include repeated transport of the matrix and solid particles between two standard syringes coupled with a fitting. In a further embodiment, this fitting includes a static mixing element. In additional embodiments, the physical mixing is accomplished in a pouch. In still further embodiments, the organogel matrix and particles of the active agent are added to a container with a mixing element, for example a mixing system used to form bone cement such as a PMMA mixing system. Particular examples include the DePuy Spine CONFIDENCE Spinal Cement Mixer or TRAUMACEM V+ Mixer. In a further embodiment, the container with a mixing element has the form and function of a syringe, such as Sulzer Medmix's F-system or P-system syringes.

According to certain embodiments, the organogel matrix has a solubility in water of less than 1g/L.

In certain embodiments, the organogel matrix is configured to adhere to the soft tissue, hard tissue, or both, in a substantially aqueous environment

The organogel composition comprises solid particles of a water-soluble antimicrobial or anti-infective agent, which may be an antibiotic agent. In certain embodiments, the antibiotic agent is gentamicin, moxifloxacin, or vancomycin. In additional embodiments, the active agent is soluble, freely soluble, or very soluble in water. According to alternative embodiments, the active agent is sparingly soluble, slightly soluble, very slightly soluble, or insoluble in water. In still further embodiments, the active agent solid particles have a D(50) median particle size distribution in the range of 1 µm to about 1 mm.

According to certain embodiments, the organogel matrix further comprises one or more excipients. In certain embodiments, the one or more excipients includes biocompatible surfactants or biocompatible hydrophilic small molecules, or a combination thereof. In still further embodiments, the one or more excipients includes Poly(ethylene glycol) (PEG), Pluronic F127, Tween 80, Tween 60, Tween 20, or a mixture of any combination thereof.

According to certain embodiments, the contemporaneous compounding and delivering are within 1.5 hours or less of each other. In still further embodiments, the contemporaneous compounding and delivering are within 1.0 hours or less, and can be within 0.5 hours or less.

### EXAMPLES

### Reference Example 1: Metal Adherence

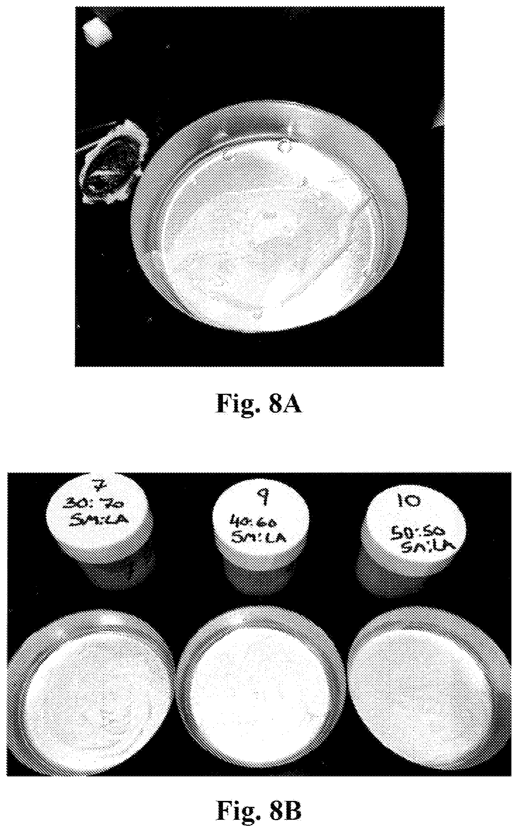

An application of 50:50 sorbitan monostearate:linoleic acid organogel matrix was applied onto the bottom surface of a metal weigh boat through an aqueous medium of phosphate buffered saline (PBS), as shown in Fig. 8A. The organogel matrix exhibited good adherence to the metal surface of the weigh boat through the aqueous medium of the PBS.

In a separate experiment, three different organogel matrix formulations were applied to the bottom surface of a metal weigh boat. The organogel formulations were composed of 30:70, 40:60, and 50:50 sorbitan monostearate:linoleic acid respectively. Each formulation was forcefully rinsed with deionized water from a squirt bottle to simulate aqueous conditions and fluid flow that can occur *in vivo.* The water stream did not dislodge the 40:60 and 50:50 organogel matrix formulations, while some of the 30:70 sorbitan monostearate: linoleic acid organogel matrix was dislodged but a visually-apparent quantity remained, which can be seen in Fig. 8B.

These results indicate the organogel matrix formulations of the present disclosure can be applied to metal surfaces, such as implantable medical devices like orthopedic implants in wet environments. Thus, the methods described herein can permit the organogel drug depots to be applied to the implantable medical device *in vivo* after completion of internal fixation, as well as prior to or subsequent to final irrigation before closure of the orthopedic implant site. It is further noted that the solid/semisolid state of the organogel matrix at the time of delivery is sufficiently important to prevent the migration of the matrix away from the intended site and achieve good adherence to the desired surface.

### Reference Example 2: Ex vivo application to simulated orthopedic implant site

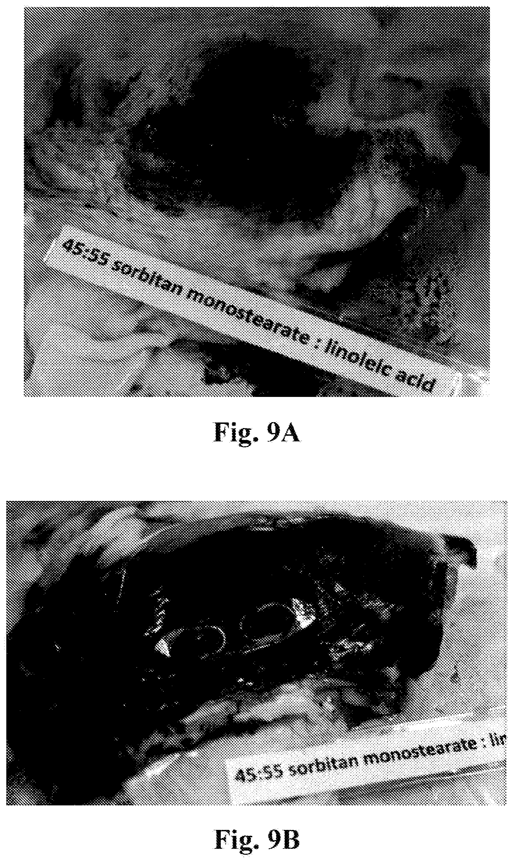

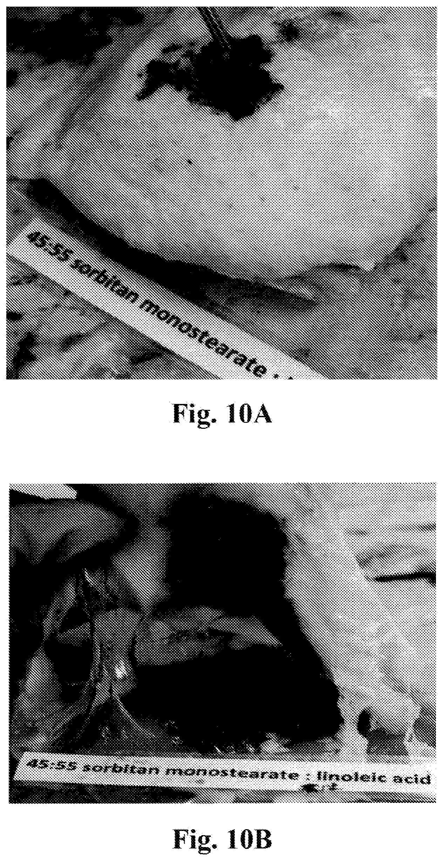

A 45:55 sorbitan monostearate:linoleic acid organogel matrix was loaded with toluidine blue O dye (to simulate a hydrophilic active agent) and was applied as a simulated organogel drug depot to orthopedic implant sites on chicken thighs. One site was used for open application along with a stainless-steel plate, as shown in Figs. 9A-B. A second site was used for percutaneous injection of the organogel drug depot at the simulated orthopedic implant site, as shown in Figs. 10A-B. In open application of the organogel (Figs. 9A-B), it was noted that the organogel matrix was adherent to the hypodermis-contaminated stainless-steel plate, the muscle fascia, and the hypodermis even after irrigation with saline and manual rubbing of the site. The percutaneous simulated surgical site (Figs. 10A-B) demonstrated the ability to cover a 40 cm² area through a single incision with adhesion to both hypodermis and muscle fascia.

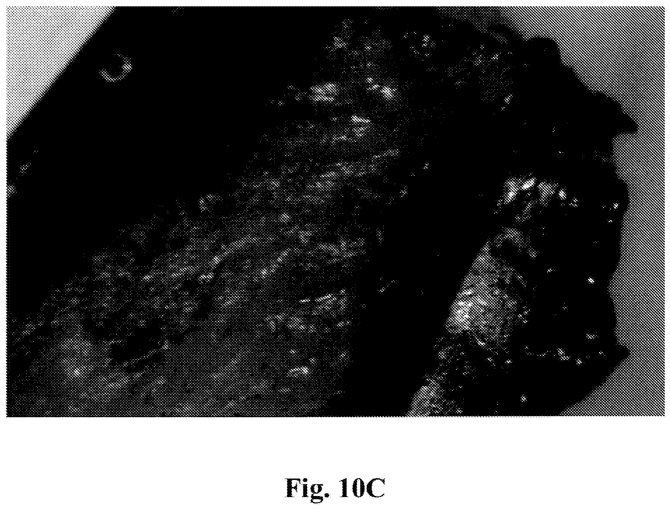

It is believed that the semisolid nature of the organogel matrix permits it to be sheared over a large area without compromising the overall matrix; without being bound to any particular theory, this can be facilitated by weak associations between particles or self-assembled structures that stabilize the semisolid. The semisolid nature of the organogel matrix appears to prevent penetration of the matrix into adjacent tissue structures, as shown in Fig. 10C (noting that the organogel adheres to the fascia of the muscle but does not penetrate the muscle), while permitting the eluted drug to effectively release from the matrix and penetrate the adjacent tissue. Such results demonstrate the ability of the organogel matrix - and by extension, the organogel drug depot - to be both irrigation and migration resistant when subject to simulated *in vivo* conditions.

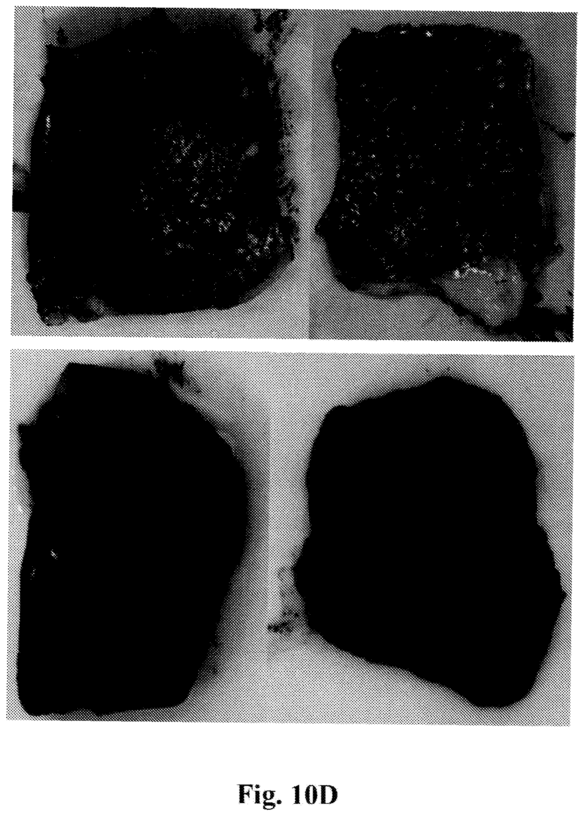

In a further experiment, pieces of the chicken thigh tissue that had been covered with the organogel drug depot (muscle fascia, see Fig. 10D top left, hypodermis, see Fig. 10D top right) were examined for release of the toluidine blue O dye (representing hydrophilic active agent particles) from the chicken thigh tissue. Two coated pieces of chicken thigh tissue were submerged in containers holding phosphate buffered saline (PBS). The PBS was exchanged hourly for four hours. The experiment showed that the organogel matrix continued to adhere to the chicken thigh tissue and did not penetrate into the muscle tissue or fascia, further supporting the migration resistant nature of the material. However, toluidine blue O dye was released into the buffer at each time point, and the released toluidine blue O dye penetrated both the muscle and skin tissue (see bottom Fig. 10D).

### Reference Example 3: Melt Reconstitution

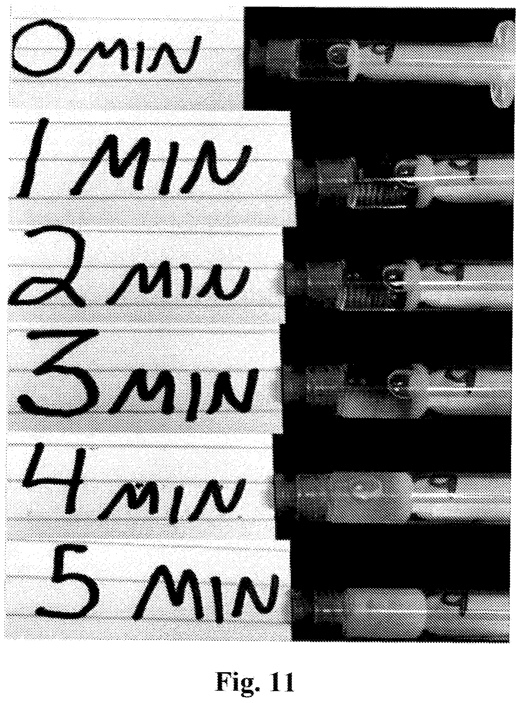

An organogel matrix formulation of 45:55 sorbitan monostearate:linoleic acid was prepared and heated to achieve a molten state. The molten organogel matrix was loaded into a syringe and allowed to cool to room temperature. Its appearance was observed at one-minute intervals until the matrix was visually observed to reform into a solid/semisolid state. As shown in Fig. 11, the organogel matrix returned to a solid/semisolid state within approximately 5 minutes.

### Reference Example 4: Heat Energy Analysis

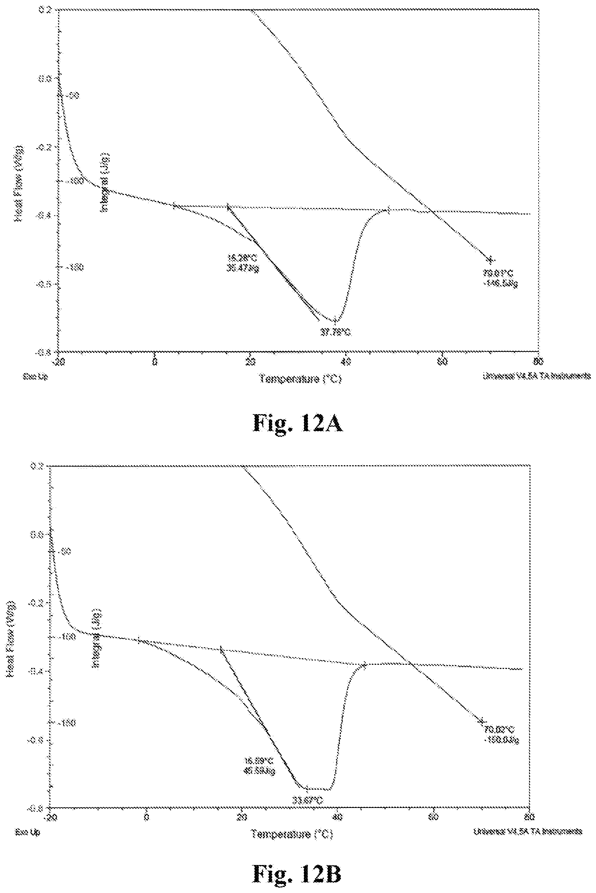

In order to determine the total amount of heat required to transform the organogel matrix into a molten state, two organogel matrix formulations were prepared; one, a base formulation of 45:55 sorbitan monostearate:linoleic acid, and a second including the base formulation with the addition of excipients, 5% PEG₁₀ₖ 0.5% Pluronic F127. Each sample was measured in a differential scanning calorimeter (DSC) from -20°C to 80°C. The resultant graphs of the scans are shown in Figs 12A-B, respectively. The results indicate that from room temperature (approx. 20°C) to above melting temperature (approx. 70°C) requires about 150 J/g. This value is well within the limits produced by commercially available battery powered heaters, and which can be utilized, for example, in the heating devices as shown and described herein.

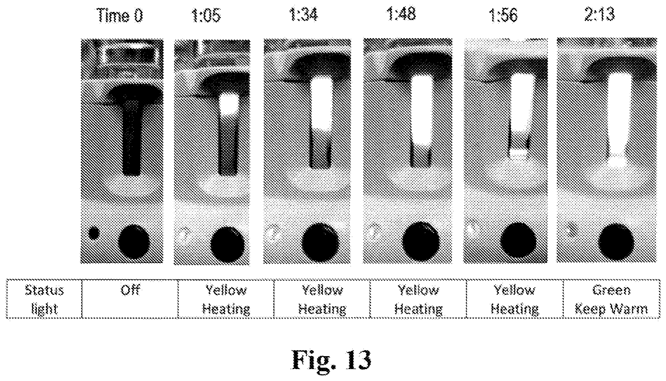

As an example, a battery-powered microprocessor-controlled device according to the embodiment shown in Fig. 7 was utilized to melt 6 grams of 45:55 sorbitan monostearate:linoleic acid with 5% PEG₁₀ₖ and 0.5% Pluronic F127. As can be seen in Fig. 13., the melting chamber was backlit, permitting visual observation through container 35 of melting as indicated by light passing through the container 35 holding the molten organogel matrix. Full melt was achieved in approximately 2 minutes. Thermal control is not limited to microprocessor control, and could be achieved through a variety of means, including, but not limited to, electromechanical thermostats, electronic thermostats, or the use of positive temperature coefficient heating elements. Alternatively, the heating could be achieved through exothermic chemical reaction including, but not limited to, the oxidation of pure iron to iron oxide.

### Example 5: Method for in vitro elution from organogel gentamicin formulations

To evaluate the in vitro release of gentamicin sulfate from organogel formulations, approximately 193 mg of organogel-gentamicin sulfate formulation was loaded into a 13 mm diameter depression in a stainless-steel disc and placed in a jar with 60 mL of phosphate buffered saline at 37°C. The buffer was sampled at 1 hour, and 1, 2, 3, 4, 7, 10 and 14 days. Complete buffer exchange was performed at all timepoints except 1 hour. Each eluent sample was briefly vortexed to ensure the sample was homogenous. Then, 1 mL of each eluent sample and corresponding blank was transferred to a separate 15 mL sterile tube. An equal volume of ethyl acetate was then added to each tube and then the tubes were either vortexed or manually shaken for about 10 seconds. The tubes were then placed on a test-tube rack and the layers were allowed to separate undisturbed for 10 -15 minutes. The top layer containing any organogel components dissolved in the ethyl acetate layer was then carefully removed with a micropipette tip. An additional volume of ethyl acetate was then added to the tube and the extraction was repeated again to remove any additional organogel or excipients from the aqueous layer. The extracted aqueous bottom layer that contained gentamicin sulfate was then derivatized for quantification by UV absorbance. The derivatization reaction involved the reaction of the three primary amine groups on gentamicin with o-phthaladehyde (OPA) under basic conditions to form UV-absorbing fluorophores. Briefly, 1 mL of either the blank (usually phosphate buffered saline (PBS)) or extracted sample was added to a 15 mL sterile tube. To this, 500 µL isopropyl alcohol (IPA) and 150 µL of basic OPA was added to each tube that was then vortexed to mix. The tubes were then covered with foil for 15 minutes to allow the derivatization reaction to proceed at room temperature. Each sample was then transferred to a disposable plastic cuvette and the absorbance of the sample and blank was measured on a spectrophotometer at 332 nm. Quantification of gentamicin sulfate was then determined by interpolation from a standard curve constructed with gentamicin standards using Beer's law.

### Reference Example 6: In vitro elution from syringe-to-syringe mixed organogel formulations

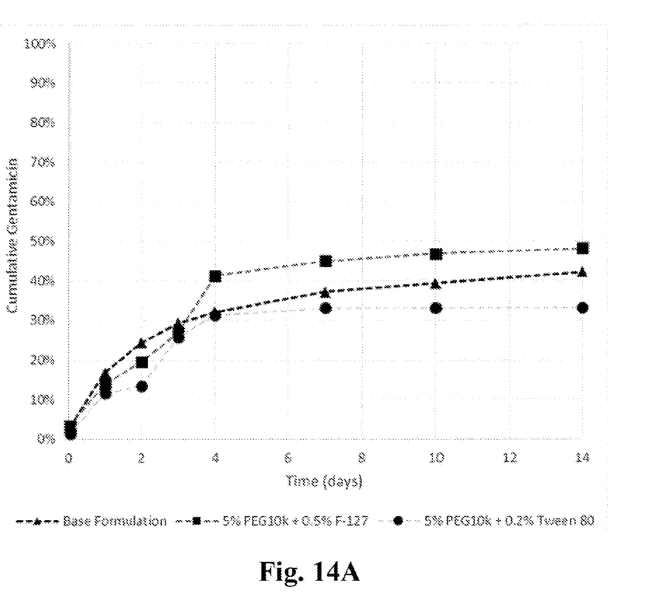

A 3 mL syringe of organogel formulation was loaded with approximately 930 mg of organogel formulation and a second syringe was loaded with micronized gentamicin sulfate equaling 20% of the organogel mass, approximately 187 mg. The micronized gentamicin sulfate was blended into the organogel by syringe-to-syringe mixing at room temperature. The organogel formulations consisted of a 45:55 sorbitan monostearate:linoleic acid base formulation and two additional formulations that included the base formulation plus excipients. One excipient formulation included a 5% PEG₁₀ₖ and 0.5% Pluronic F-127 excipient addition, and a second excipient formulation included 5% PEG₁₀ₖ and 0.2% Tween 80 excipient addition. The mixed formulations contained 16.7% gentamicin sulfate by mass. Figure 14A illustrates the in vitro release of gentamicin sulfate from the organogel formulations with syringe-to-syringe mixing at room temperature. In the first day, 4-5 mg of gentamicin sulfate (12-17%) was released from the organogel-gentamicin sulfate formulations with 8-9 mg (26-29%) released through day 3. A lower rate of release was observed from day 4 through day 14, reaching a total percent observed in the buffer of approximately 41% cumulative gentamicin sulfate. Of note, the release of hydrophilic gentamicin sulfate from the organogel formulations was controlled without noted burst release; gentamicin sulfate release at 1 hour was between 0.4 and 1.1 mg (1-3%).

### Reference Example 7: In vitro elution from melt-mixed organogel formulations

A 3 mL syringe of organogel formulation was loaded with approximately 947 mg of grease formulation and a glass vial was loaded with micronized gentamicin sulfate equaling 20% of the organogel mass, approximately 189 mg. The organogel formulation was injected into the glass vial using a vial adapter. The vial was placed into a water bath to melt the organogel. The vial was then shaken to suspend the gentamicin sulfate particles in the molten organogel, and the organogel plus gentamicin sulfate was drawn back into the syringe to cool and form into semisolid formulations of organogel plus gentamicin sulfate. The melt-mixed formulations contained 16.7% gentamicin sulfate by mass. As above, the organogel formulations consisted of a 45:55 sorbitan monostearate:linoleic acid base formulation and same two excipient formulations, base formulation plus 5% PEG₁₀ₖ and 0.5% Pluronic F-127 and base formulation plus 5% PEG₁₀ₖ and 0.2% Tween 80.

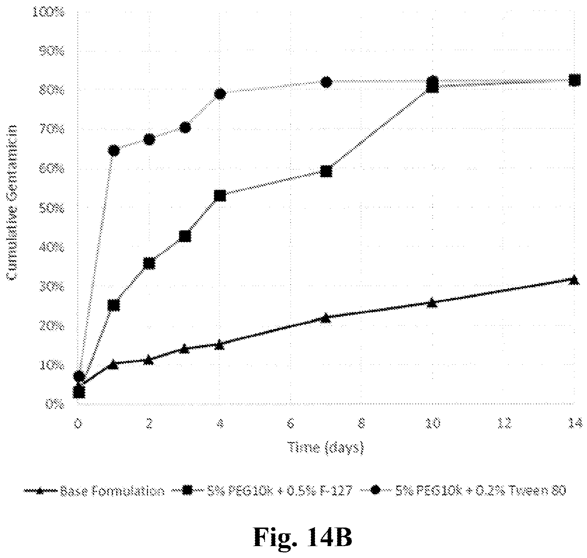

Figure 14B illustrates in vitro release of gentamicin sulfate from melt-mixed organogel formulations. The use of melt-mixing enabled a range of gentamicin sulfate release rates from organogel formulations. In the first day, the base formulation released 3.3 mg (10%) of its gentamicin sulfate, while the excipient formulations released 8.2 mg (25%) and 20.8 mg (65%) gentamicin sulfate in one day. As above, no notable burst release was observed with 3-7% gentamicin sulfate release in one hour. The base formulation released 32% of its gentamicin sulfate load in a linear fashion over 2 weeks. The 5% PEG + 0.5% F-127 formulation released 53% of its gentamicin in 4 days, and 81% within 10 days. The 5% PEG + 0.2% Tween 80 formulation released 65% of its gentamicin sulfate in the first day and 79% by 4 days. The release curves of Fig 14B demonstrate the ability to "tune" the organogel matrix by blending with excipients that increase water penetration into the matrix and dissolution of the therapeutic molecule and matrix. The melt-mixed formulations provided a greater range of release rates, with lower cumulative release of gentamicin sulfate from the base formulation in the melt-mixed form versus the room temperature mixed example, while simultaneously demonstrating faster release of the gentamicin sulfate from the excipient formulations in the melt-mixed examples versus the room temperature mixed examples.

### Example 8: Organogel v. Hydrogel in vitro antibiotic elution

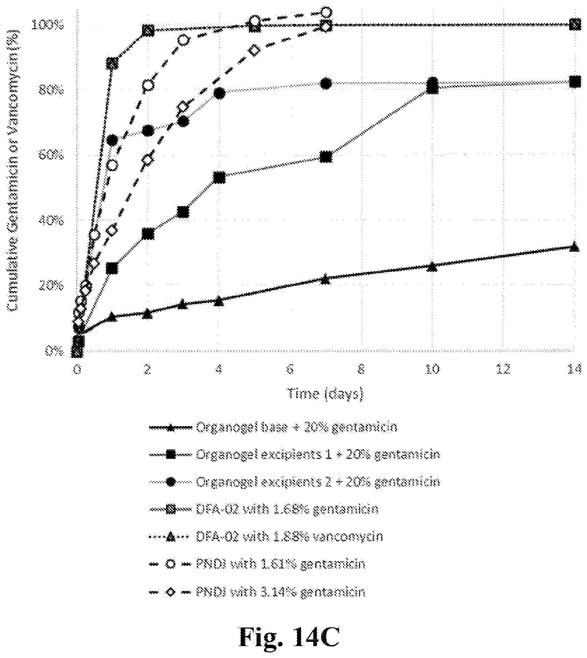

Gentamicin sulfate release from the three melt-mixed organogel formulations described above and shown in Fig. 14B were compared against published literature values for several hydrogel drug depots. Release data was available for the following hydrogel drug depots: Dr. Reddy's DFA-02 formulated with 1.68% gentamicin plus 1.88% vancomycin (Penn-Barwell JG, Murray CK, and Wenke JC, J Orthop Trauma 2014; 28:370-375) and Sonoran Biosciences PNDJ formulated with either 1.61% gentamicin or 3.14% gentamicin (Overstreet D, McLaren A, Calara F, Vernon B, and McLemore R, Clin Orthop Relat Res 2015; 473:337-347). As shown in the graph in Fig. 14C, the release of gentamicin and vancomycin from Dr. Reddy's DFA-02 was 88% complete in the first day and 98% complete by day 2. Sonoran's PNDJ formulations took 5-7 days to reach approximately 100% release, with 59% or 81% released by day 2. In contrast, the base organogel formulation released only 11% of its gentamicin by day 2 and 22% in the first week. The addition of excipients was able to bring the two-day release to either 36% or 68%. This comparison demonstrates that organogels may achieve greater duration of drug release than achieved with hydrogels, and release rates are tunable by the selection of appropriate excipients.

### Reference Example 9: Hydrophobic v hydrophilic in vitro elution profiles

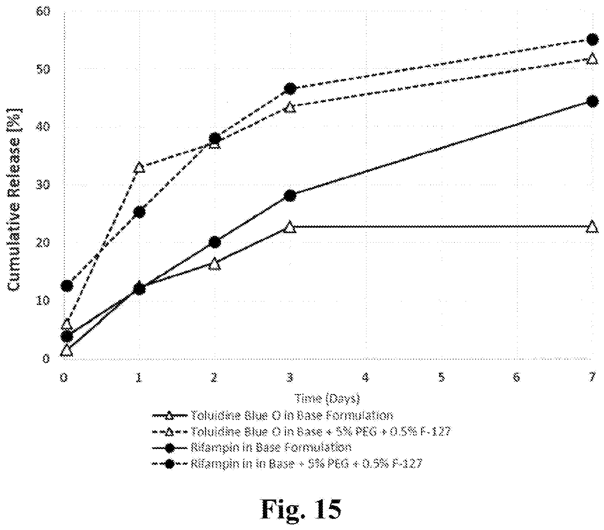

Two organogel matrix base formulations having 45:55 sorbitan monostearate:linoleic acid compositions were prepared by physical syringe-to-syringe mixing at room temperature in the semisolid state. One organogel matrix formulation included a 10% by weight addition of toluidine blue O dye to simulate a hydrophilic active agent. The other organogel matrix formulation included 10% by weight of rifampin, a relatively more hydrophobic active agent. Two additional organogel matrix excipient formulations were prepared with the base formulations previously described and including the addition of 5% PEG₁₀ₖ and 0.5% Pluronic F-127. The formulations were then placed into a 13 mm diameter depression in a stainless-steel disc and placed in a jar with 60 mL of PBS plus 20% fetal bovine serum at 37°C, and their respective active agent elution profiles were measured. At each time point, the color of eluent was compared to visual standards prepared of 0, 1, 2.5, 3.75, 5, 7.5, 10, 15, 20, 30, 40, and 50 ppm of rifampin or toluidine blue O dye in PBS plus 20% fetal bovine serum. As shown in the graph at Fig. 15, each pair (i.e., base and excipient formulations) of organogel drug depots released their active agents at similar rates. In the first 3 days, the excipient-containing formulations eluted approximately 45% of their active agents, while the base formulations eluted approximately 25% of their active agents. At 7 days, both excipient formulations eluted approximately 53% of their active agents, while there was a deviation between the release of rifampin and Toluidine Blue O between days 3 and 7 in the base formulation. The rifampin sample reached 44%, while the toluidine blue O sample remained at 23%. Thus, it can be seen the organogel matrix formulations can elute two dissimilar active agents at similar rates over a one-week period into serum-containing buffer.

Furthermore, because the organogel matrix of the present disclosure has sparing water solubility due to the hydrophobic nature of its composition, the active agent particles' elution is limited by water availability for dissolution (irrespective of either a hydrophilic or hydrophobic active agent), followed by diffusion through the hydrophobic matrix. As previously discussed above, significant disadvantages are associated with hydrogel drug depots such as DAC-Gel, Dr. Reddy's DFA-02, Sonoran PNDJ, and Poloxamer 407 thermoreversible hydrogels. These exemplary hydrophilic drug depots are water-rich environments where the drug is in its soluble form, and release is only limited by diffusion through the water-rich network. As a result, hydrogel matrices are unable to achieve the long release durations and high drug loading ratios of the organogel matrices described herein. An additional benefit of the limited water availability within the organogel matrix is the relative stability of the active agent within the depot. Where the active agent is in particulate form, it has limited susceptibility to chemical reactions associated with degradation. Furthermore, the dissolution-limited approach enables both hydrophobic and hydrophilic molecules to be released at similar rates.

### Reference Example 10: Antibacterial efficacy versus Staphylococcus aureus biofilm

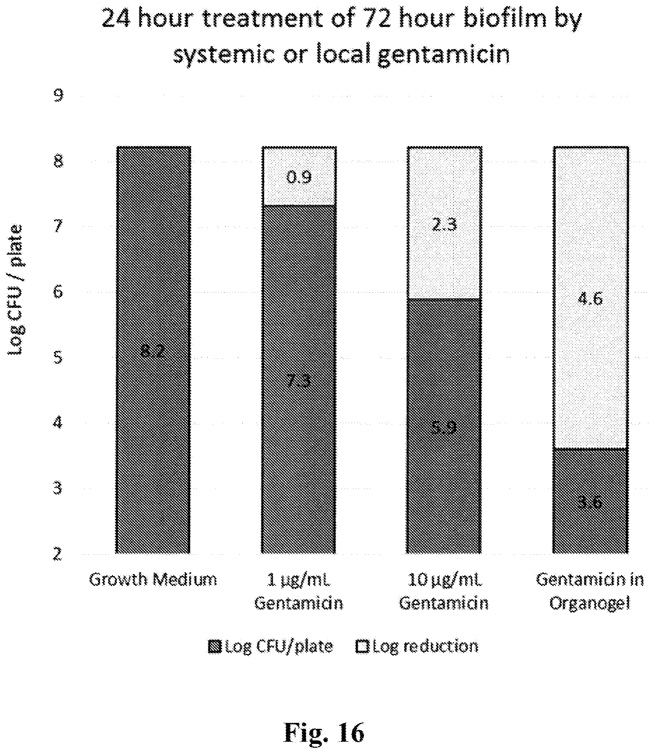

Four sets of standard stainless steel trauma plates were colonized with *Staphylococcus aureus* while rolling in an inoculum of 10⁵ CFU/mL in 0.3% tryptic soy broth (TSB) in 15 mL tubes over 4 hours. The inoculated plates were placed into a lateral flow cell with intermittent 0.3% TSB medium replenishment every 4 hours with no flow between feedings. Biofilm growth proceeded in 0.3% TSB medium at 37°C for 3 days to produce a mature biofilm. Each plate was rinsed twice in PBS, then returned to a sterile lateral flow cell for 1 day of treatment. One set of plates served as a control group, fed with 0.3% TSB growth medium. The second set was treated with 0.3% TSB plus 1 µg/ml gentamicin sulfate. The third set was treated with 0.3% TSB plus 10 µg/ml gentamicin sulfate. These concentrations represent a range of clinically-relevant blood levels for systemic administration of gentamicin sulfate, here provided as a supplement to the 0.3% TSB medium. The fourth group consisted of a 590 mg organogel drug depot placed into the growth chamber without contacting the trauma plate with adhered bacterial biofilm. The organogel drug depot included 16.7% by weight of gentamicin sulfate melt-mixed with 45:55 sorbitan monostearate:linoleic acid (corresponding to a 1:5 weight ratio of drug: organogel matrix) with the addition of 5% PEG₁₀ₖ and 0.5% Pluronic F-127 as excipients. This group was fed with 0.3% TSB growth medium without any antibiotics. In all four sets, the culture medium was exchanged once every four hours by lateral flow for four minutes. Note that the gentamicin sulfate released from the organogel formulation inside the growth chamber was rinsed away every four hours, requiring additional gentamicin sulfate to elute from the formulation to continue antibacterial activity. As shown in Fig. 16, gentamicin sulfate released from the organogel drug depot was more effective against a 3-day *S. aureus* biofilm grown on a trauma plate than systemic delivery of gentamicin sulfate. Importantly, even though the second and third sets of implants were continuously exposed to clinically-relevant concentrations of gentamicin sulfate over 24 hours, the organogel drug depot showed higher effectiveness in killing bacteria in the biofilm despite the gentamicin sulfate being rinsed away every four hours.

### Reference Example 11: Organogel matrix melting temperature by vial inversion

Six organogel matrix formulations were prepared by dissolving sorbitan monostearate, sodium stearate, or combinations thereof in linoleic acid at elevated temperature (79.3°C for sorbitan monostearate formulations or 146.0°C for formulations containing sodium stearate), then cooling to room temperature with shear provided by a vortexing device. Two formulations containing hydrophilic excipients of PEG₁₀ₖ and Pluronic F-127 were prepared. The six organogel matrix formulations in 20 mL glass scintillation vials were inverted in a metal vial rack and placed in a forced air oven with a calibrated thermocouple positioned between the two central vials. The oven temperature was increased in 2°C increments and held for a minimum of 10 minutes at each temperature. Between each temperature increment the samples were observed for their ability to support their own mass or whether they dropped. The melting temperature range recorded was the range between the highest temperature of observed self-supporting matrix and the first temperature observed where the matrix dropped to the bottom of the vial. The inclusion of sodium stearate increased the formulation melting temperature in a dose-dependent manner. 45:55 sorbitan monostearate:linoleic acid formulations melted from 43.1°C - 44.7°C, but 35:65 and 45:55 sodium stearate:linoleic acid formulations melted from 62.7°C - 68.5°C and 84.3°C - 86.2°C, respectively. The blended formulation of 30:15:55 sodium stearate:sorbitan monostearate: linoleic acid melted from 62.7°C - 64.6°C. The inclusion of hydrophilic excipients did not appreciably affect the melting point. The inclusion of a high-melting organogelator, such as sodium stearate was advantageous in raising the melting temperature so that the product would remain in a semisolid state in hot environments.

### Reference Example 12: Melting point of organogel matrix with fatty acid ester organogelators varies by biocompatible organic solvent

Organogel matrix formulations based upon sorbitan monostearate or glyceryl monostearate were prepared at organogelator:solvent ratios from 25:75 to 45:55 in various oils by melting at 80°C, removing from the oven and cooling with shear provided by a vortexing device. Differential scanning calorimetry (DSC) was used to determine the melting point of the samples at a heating rate of 10°C/minute. The melting peak temperature and melting endset temperatures were determined using TA Instruments software. The melting endset was defined as the intersection of the melting peak's second tangent line with the baseline of the melting peak. The melting temperature of sorbitan monostearate organogel matrix formulations varied by concentration in glyceryl caprylocaprate but not in soybean oil. The melting point of the soybean oil formulations was within the published melting point of sorbitan monostearate (50-60°C, Handbook of Pharmaceutical Excipients, 6th Edition, 2009), while glyceryl caprylocaprate formulations melted significantly lower (T_{m,peak} 32.8-37.7°C). Glyceryl monostearate organogel matrix formulations in soybean oil or cottonseed oil melted within the gelator's published melting point range (55-60°C, Handbook of Pharmaceutical Excipients, 6th Edition, 2009), while formulations prepared in DL-alpha-tocopherol acetate, ethyl oleate, and glyceryl monocaprylocaprate melted below that range. A 40:60 glyceryl monostearate: castor oil sample had a particularly broad melting peak with a melting endset of 56.0°C, approaching the 57.6°C and 57.5°C observed in cottonseed oil and soybean oil. The data demonstrate that semisolid formulations prepared from a single gelator have melting points that vary by chosen biocompatible organic solvent. Without being bound by any particular theory, the melting point of semisolid formulations of a given organogelator can be maximized where a solvent is selected such that the organogelator is incompletely dissolved at the organogelator's melt temperature and the molten organogelator is miscible with the biocompatible organic solvent. Upon cooling, a semisolid formulation is obtained.

**Table 1: Melting point of sorbitan monostearate and glyceryl monostearate organogel matrix formulations prepared in various biocompatible organic solvents**

| Organogel matrix formulation | T_{m,peak} °C | T_{m,endset} °C |
|---|---|---|
| 35:65 sorbitan monostearate: ethyl oleate | 45.0 | 48.5 |
| 45:55 sorbitan monostearate: ethyl oleate | 45.4 | 48.2 |
| 25:75 sorbitan monostearate:glyceryl monocaprylocaprate | 32.8 | 37.2 |
| 35:65 sorbitan monostearate: glyceryl monocaprylocaprate | 35.4 | 39.0 |
| 45:55 sorbitan monostearate: glyceryl monocaprylocaprate | 37.7 | 41.3 |
| 25:75 sorbitan monostearate:soybean oil | 53.0 | 56.5 |
| 35:65 sorbitan monostearate:soybean oil | 53.0 | 57.1 |
| 45:55 sorbitan monostearate:soybean oil | 53.0 | 56.4 |
| 40:60 glyceryl monostearate: DL-alpha-tocopherol acetate | 49.5 | 52.4 |
| 40:60 glyceryl monostearate:castor oil | 47.8 | 56.0 |
| 40:60 glyceryl monostearate: cottonseed oil | 55.6 | 57.6 |
| 40:60 glyceryl monostearate:ethyl oleate | 47.5 | 49.8 |
| 40:60 glyceryl monostearate:glyceryl monocaprylocaprate | 37.5 | 49.1 |
| 40:60 glyceryl monostearate:soybean oil | 55.5 | 57.5 |

### Example 13: Examples of high-melting formulations, their melting temperature, and syringe expression force

Example formulations were fabricated by melting or dissolving the organogelator(s) in the selected base biocompatible organic solvent(s) in a glass vial at 110°C-160°C, cooling the materials in a syringe, equilibrating the formulations at room temperature, shearing the formulations by mixing between two coupled 5 mL NormJect Syringes, and allowing to equilibrate again prior to evaluation. Formulation melting point was characterized by DSC using a 10°C/minute heating rate and analyzing the first heat. The formulations were further characterized by measuring syringe expression force from a 5 mL NormJect syringe at 1 mm/second using an MTS Corp test system. Data was recorded at 24 Hz.

### Example 13-1: Examples of high-melting formulations, their melting temperature, and syringe expression force grouped by formulation family

Formulation family #4 consisted of room temperature blending of two formed gels: sodium stearate in glyceryl monocaprylocaprate organogel blended with sorbitan monostearate in a second biocompatible organic solvent. Formulations were characterized for melt temperature by DSC using a 10°C/minute heating rate from 5°C to 120°C to -40°C to 120°C. A single formulation that did not melt by 120°C was additionally heated from 40°C to 180°C at 10°C/minute. Syringe expression data was collected using a 100N load cell. The peak syringe expression force and the average syringe expression force were reported as mean ± standard deviation across three samples. The average syringe expression force for each sample was calculated as the average force recorded at each datapoint once a plateau was reach on the force-displacement curve.

**Table 2: Example organogel matrix formulations grouped by family, their melting point by DSC, and syringe expression force.**

| **Family** | **Organogel matrix formulation** | **Formulation melting point by DSC** | | **Peak expression force, lbf** | **Average expression force, lbf** |
|---|---|---|---|---|---|
| | | **Peak, °C** | **Endset, °C** | | |
| 1 | 25:75 Sodium Stearate: Glyceryl Monocaprylocaprate | 102.0 | 106.5 | 6.52 ± 1.54 | 5.66 ± 1.03 |
| 2 | 10:10:80 Sodium Stearate: Sorbitan Monostearate: Glyceryl Monocaprylocaprate | 64.5 | 80.2 | 5.88 ± 0.65 | 5.41 ± 0.72 |
| | 12:6:82 Sodium Stearate: Sorbitan Monostearate: Glyceryl Monocaprylocaprate | 71.1 | 80.4 | No Data | No Data |
| 3 | 20:5:75 Sodium Stearate: Stearic Acid: Glyceryl Monocaprylocaprate | 103.0 | 105.7 | 6.98 ± 0.69 | 5.98 ± 0.55 |
| 4 | 12.5:37.5 Sodium Stearate: Glyceryl Monocaprylocaprate / 20:30 Sorbitan Monostearate: Castor Oil | 107.2 | 111.0 | 3.54 ± 0.24 | 3.25 ± 0.13 |
| | 12.5:37.5 Sodium Stearate: Glyceryl Monocaprylocaprate / 20:30 Sorbitan Monostearate: Soybean Oil | 104.6 | 109.0 | 3.48 ± 0.24 | 3.08 ± 0.49 |
| | 12.5:37.5 Sodium Stearate: Glyceryl Monocaprylocaprate / 20:30 Sorbitan Monostearate: Medium-chain Triglycerides | 101.8 | 108.9 | 3.72 ± 0.89 | 3.31 ± 0.46 |
| 5 | 20:80 12-Hydroxystearic Acid: Castor oil | 59.1 | 65.3 | 4.54 ± 0.49 | 4.26 ± 0.49 |
| | 20:80 12-Hydroxystearic Acid: Soybean oil | 73.5 | 76.7 | 4.74 ± 0.11 | 4.20 ± 0.28 |
| 6 | 20:80 Hydrogenated Castor Oil: Castor Oil | 75.3 | 78.3 | 2.44 ± 0.16 | 2.21 ± 0.13 |
| | 30:70 Hydrogenated Castor Oil: Medium-chain triglycerides | 76.8 | 80.3 | 5.04 ± 0.84 | 4.75 ± 0.69 |
| | 30:70 Hydrogenated Castor Oil: Soybean Oil | 77.7 | 84.4 | 2.61 ± 0.34 | 2.35 ± 0.34 |
| 7 | 35:65 Hydrogenated Phosphatidylcholine from Soy: Soybean Oil | 101.6 | 112.8 | 2.06 ± 0.24 | 1.82 ± 0.19 |
| | 30:70 Hydrogenated Phosphatidylcholine from Soy: Castor Oil | 82.8 | 85.5 | 1.65 ± 0.08 | 1.33 ± 0.15 |
| 8 | 30:70 Cholesterol: Castor oil | 133.2 | 141.5 | 1.96 ± 0.11 | 1.74 ± 0.09 |
| | 20:10:70 Cholesterol: Glyceryl Distearate: Soybean oil | 90.9 | 108.3 | 13.98 ± 1.42 | 6.63 ± 0.83 |

The formulation families listed in Table 2 can be defined as follows:
1) Sodium stearate or sodium oleate in a biocompatible organic solvent at various concentrations (provided for reference);
2) Sodium stearate or sodium oleate plus a fatty acid ester in a biocompatible organic solvent (provided for reference);
3) Sodium stearate and stearic acid or sodium oleate and oleic acid as organogelators in glyceryl monocaprylocaprate (provided for reference). In the present example, sodium oleate did not dissolve in soybean oil, cottonseed oil, or castor oil at up to 160°C; temperatures above 200°C were required for dissolution in castor oil or cottonseed oil;
4) Sodium stearate or sodium oleate in glyceryl monocaprylocaprate blended in the semisolid state with a second gelator in a second oil also in its semisolid state (provided for reference). In this organogel matrix formulation family two separate semisolids are formed in a separate biocompatible organic solvent, such that the lipid crystals of each organogelator are formed in each biocompatible organic solvent;
5) Semisolid organogel matrix formulations based upon 12-hydroxystearic acid as an organogelator in various biocompatible organic solvents at various concentrations (provided for reference);
6) Semisolid organogel matrix formulations based upon hydrogenated castor oil as an organogelator in various biocompatible organic solvents at various concentrations (provided for reference);
7) Semisolid organogel matrix formulations based upon hydrogenated soy phosphatidylcholine with or without further viscosupplementation (provided for reference);
8) Semisolid organogel matrix formulations based upon cholesterol(s) with or without further viscosupplementation.

Each formulation family described above demonstrates the capability to produce a high melting organogel matrix that can be expressed from a standard luer lock syringe. Any of the above formulations can be altered with further excipients to modulate the release properties of an included drug or to affect handling properties for spreading onto tissue. Expected excipient classes include hydrophilic polymers, such as polyethylene glycol of molecular weight from 400 to 20,000 Daltons, and surfactants, such as polysorbate 20, polysorbate 60, polysorbate 80, or Pluronic F-127, for example.

### Example 13-2: Melting point of example organogel matrix formulations containing sodium oleate

Table 3 presents organogel matrix formulations fabricated from sodium oleate by dissolution in biocompatible organic solvents at elevated temperature and cooling to form a gel. Sodium oleate-based formulations melt temperatures data, shown below in Table 3, show that the formulations all melted above 71°C. As such, sodium oleate-based formulations can be considered suitable for high-melting formulations such as described above in Table 2.

**Table 3: Example sodium oleate formulations (provided for reference)and their melting points by DSC**

| **Organogel matrix formulation** | **Formulation melting point by DSC** | |
|---|---|---|
| | **Peak, °C** | **End Set, °C** |
| 20:80 sodium oleate: DL-alpha-tocopherol acetate | 105.5 | 117.9 |
| 20:80 sodium oleate: castor oil | 116.3 | 117.9 |
| 20:80 sodium oleate: cottonseed oil | 112.8 | 116.6 |
| 25:75 sodium oleate: glyceryl monocaprylocaprate | 107.1 | 115.1 |
| 20:70:10 sodium oleate:ethyl oleate:glyceryl monocaprylocaprate | 98.9 | 102.6 |
| 20:60:20 sodium oleate:ethyl oleate: glyceryl monocaprylocaprate | 97.0 | 101.8 |

### Example 13-3: Thermal characterization of example organogel matrix formulations containing cholesterol, 12-hydroxystearic acid, or hydrogenated castor oil as the primary gelator

Table 4 presents formulations prepared using cholesterol, 12-hydroxystearic acid, or hydrogenated castor oil with or without a viscosity modifier in various biocompatible organic solvents as described above. The melt temperature data shows that these gelators and various combinations can be considered high melting formulations, but that certain combinations and organic solvents can depress the melting point below the melting point of the component materials such as with 22:12:33:33 cholesterol: glycerides, mono- and di-: soybean oil: glyceryl monocaprylocaprate which melts around 41°C to 47°C. Formulating with glyceryl monocaprylocaprate and oleic acid depressed formulation melting point versus soybean oil.

The final two formulations presented in Table 4 (20:10:52:18 cholesterol: hydrogenated castor oil: glyceryl monocaprylocaprate: soybean oil, 25:15:31:29 cholesterol: glycerides, mono- and di-: soybean oil: oleic acid) were prepared as described above with some additional steps appropriate in anticipation of possible future animal study preparation. The solid ingredients were equilibrated overnight in a dry nitrogen environment (< 10% RH) prior to addition of the oil components. Prior to dissolution or melting of components, the vial headspace was purged with nitrogen. Melting and dissolution was achieved at an oven setpoint temperature of 115 °C. These formulations were filled into a 4 mL syringe which was capped and placed in a nitrogen-purged foil pouch, and subsequently gamma irradiated at 28.0-34.4 kGy.

**Table 4: Melting point of cholesterol. hydroxylated stearic acid, and hydrogenated castor oil based organogel matrix formulations prepared in various biocompatible organic solvents and/or blends.**

| **Organogel matrix formulation** | **Formulation melting point by DSC** | |
|---|---|---|
| | **Peak, °C** | **End Set, °C** |
| 20:80 12-hydroxystearic acid: medium chain triglycerides (provided for reference) | 67.2 | 70.0 |
| 20:80 hydrogenated castor oil: medium chain triglycerides (provided for reference) | 78.6 | 81.1 |
| 20:80 12-hydroxystearic acid: castor oil (provided for reference) | 59.1 | 65.3 |
| 20:80 hydrogenated castor oil: castor oil (provided for reference) | 75.3 | 78.3 |
| 22:12:66 cholesterol: hydrogenated castor oil: soybean oil | 76.8 | 80.4 |
| 22:12:66 cholesterol: glycerides, mono- and di-: soybean oil | 56.1 | 61.0 |
| 20:80 12-hydroxystearic acid: soybean oil (provided for reference) | 73.5 | 76.7 |
| 20:80 hydrogenated castor oil: soybean oil (provided for reference) | 82.0 | 84.3 |
| 22:12:33:33 cholesterol: glycerides, mono- and di-: soybean oil: glyceryl monocaprylocaprate | 41.4 | 47.1 |
| 22:12:66 cholesterol: hydrogenated castor oil: glyceryl monocaprylocaprate (provided for reference) | 61.3 | 65.6 |
| 20:80 hydrogenated castor oil: glyceryl monocaprylocaprate (provided for reference) | 67.7 | 69.8 |
| 20:80 12-hydroxystearic acid: ethyl oleate (provided for reference) | 65.3 | 67.9 |
| 20:80 hydrogenated castor oil: ethyl oleate (provided for reference) | 74.6 | 77.1 |
| 20:10:52:18 cholesterol: hydrogenated castor oil: glyceryl monocaprylocaprate: soybean oil | 62.9 | 65.9 |
| 25:15:31:29 cholesterol: glycerides, mono- and di-: soybean oil: oleic acid | 48.2 | 54.3 |

### Example 13-4: Melting point, room temperature and 4 °C syringe expression of example formulations

The syringe expression force was quantified at both room temperature and at 4 °C using the method described above and a 100N load cell. Two formulations exceeded the capacity of the 100 N load cell, and a 10 kN load cell was utilized. For chilled syringe expression, the samples were held in a refrigerator for at least 18 hours prior to evaluation, then held in an insulated cooler over wet ice. An aluminum syringe holder was chilled in a wet ice bath, placed on the test stand, the test syringe was placed into the barrel of the aluminum fixture, a calibrated thermocouple was placed between the syringe and the fixture, and syringe expression was initiated when the temperature was within 4.0±2.0°C. The observed average and standard deviation cold syringe expression temperature was 3.9±0.2°C.

Desired characteristics of the organogel formulations of the present disclosure include semisolid lipid depot with a broad thermal range, in which the lipid crystals would not melt in a desert environment, and the oil would not freeze in cold temperatures. For glyceryl monocaprylocaprate-based formulations, expression from a syringe at 4 °C required >30 lbf (Table 5); however, blending with 6% ethyl oleate reduced peak syringe expression to 4.2 lbf. Lipid crystals composed of cholesterol and hydrogenated castor oil were advantageous in obtaining high semisolid melting points.

**Table 5: DSC melting point and syringe expression force at room temperature and 4°C of example organogel formulations.**

| **Organogel matrix formulation** | **Formulation Melting by DSC** | | **RT Syringe expression** | **4°C Syringe expression** |
|---|---|---|---|---|
| | **Tₘ, Peak (°C)** | **Tₘ, End Set (°C)** | **Peak, lbf** | **Peak, lbf** |
| 19.2:17.8:63 Sodium stearate: stearic acid: glyceryl monocaprylocaprate (provided for reference) | ND | ND | 3.2 | 30.7 |
| 20.7:19.3:60 Sodium stearate: stearic acid: glyceryl monocaprylocaprate (provided for reference) | ND | ND | 4.0 | 32.3 |
| 20.7:19.3:54:6 Sodium stearate: stearic acid: glyceryl monocaprylocaprate: ethyl oleate (provided for reference) | 100.0 | 102.0 | 2.6 | 4.2 |
| 20.7:19.3:48:12 Sodium stearate: stearic acid: glyceryl monocaprylocaprate: ethyl oleate (provided for reference) | ND | ND | 2.8 | 4.6 |
| 20.7:19.3:36:24 Sodium stearate: stearic acid: glyceryl monocaprylocaprate: ethyl oleate (provided for reference) | ND | ND | 2.7 | 4.8 |
| 25:75 hydrogenated castor oil: soybean oil (provided for reference) | 77.7 | 84.2 | 2.7 | 3.3 |
| 10:16:74 hydrogenated castor oil: stearic acid: soybean oil (provided for reference) | 71.5 | 73.7 | 2.6 | 3.6 |
| 25:5:70 Hydrogenated Phosphatidylcholine from Soy: hydrogenated castor oil: castor oil (provided for reference) | 81.8 | 84.4 | 3.8 | 6.9 |
| 20:10:70 cholesterol: glyceryl distearate: soybean oil | 90.9 | 108.3 | 3.1 | 3.8 |
| 20:10:70 cholesterol: hydrogenated castor oil: soybean oil | 75.8 | 78.9 | 2.6 | 3.1 |
| 20:5:8:67 cholesterol: hydrogenated castor oil: stearic acid: soybean oil | 68.0 | 70.3 | 2.5 | 3.3 |
| 25:10:65 sodium oleate: glyceryl distearate: glyceryl monocaprylocaprate (provided for reference) | 99.7 | 103.9 | 2.1 | 4.6 |

### Example 14 - In vitro elution of moxifloxacin hydrochloride from example organogel formulations

High melting formulations from the families described in Table 2 were fabricated by melting or dissolving the organogelator(s) in the selected base biocompatible organic solvent(s) in a glass vial at 110°C-160°C, cooling the materials in a syringe, equilibrating the formulations at room temperature, shearing the formulations by mixing between two coupled 5 mL NormJect Syringes, and allowing to equilibrate again prior to evaluation. Formulation was mixed with 10% moxifloxacin hydrochloride (ChemImpex) by mass (i.e., 100mg of drug for 900mg of formulation) in a semisolid state. Mixing was performed by either by coupling two NormJect syringes, one with drug and the other with formulation, and mixing between the two back and forth 30 times, or by placing the formulation and drug into a FlackTek speedmixer and mixing for 9 minutes and 40 seconds at 2000 RPM.

### Example 14-1 In vitro elution of moxifloxacin hydrochloride from high melting organogel formulations

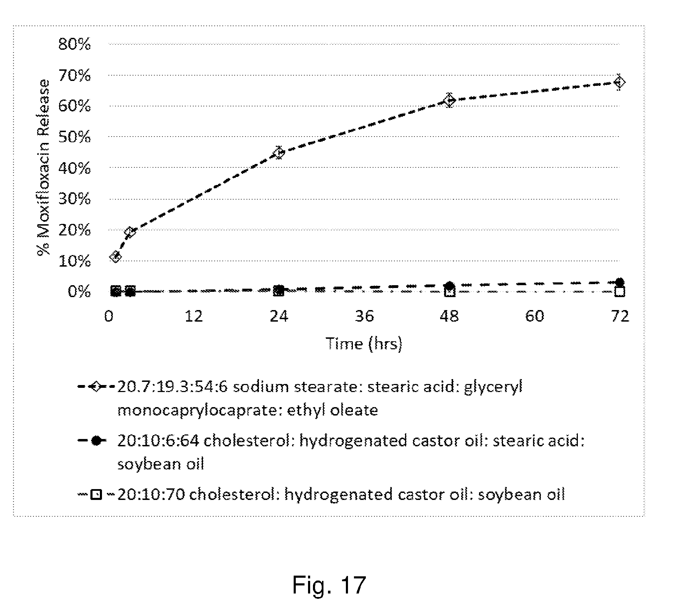

Three high melting formulations were evaluated for in vitro elution per USP<1724> "Semisolid Drug Products - Performance Tests" using a Distek USP Type 2 dissolution apparatus and Distek Model B immersion cell with 100 mesh stainless steel membrane in a 100 mL vessel with 90 mL PBS at 32°C with 50 RPM stir rate, as shown in Fig. 17. Of note, the sample thickness in a Model B immersion cell is 3 mm thick by 15mm diameter. Formulation 20:10:70 cholesterol: hydrogenated castor oil: soybean oil, referred to as formulation, was evaluated in one run, while 20.7:19.3:54:6 sodium stearate: stearic acid: glyceryl monocaprylocaprate: ethyl oleate (provided for reference) and 20:10:6:64 cholesterol: hydrogenated castor oil: stearic acid: soybean oil were evaluated in a second run under the same conditions. Each formulation was evaluated in with three replicates each. Results are displayed as mean ± standard deviation.

In the standard 3 mm sample configuration, the sodium stearate-glyceryl monocaprylocaprate-based formulation released greater than 60% of its moxifloxacin hydrochloride in 48 hours, while the soybean oil-based formulations released less than 5% moxifloxacin hydrochloride in 48 hours. The combination of sodium stearate (TCI America) and stearic acid (Kolliwax S, BASF) in glyceryl monocaprylocaprate (GMCC, CRODA) and ethyl oleate (CRODA) promoted increased cumulative drug release versus a cholesterol (Spectrum Cholesterol NF) and hydrogenated castor oil (BASF Kolliwax HCO) with or without stearic acid in soybean oil (CRODA). These findings suggested that similar sodium stearate-glyceryl monocaprylocaprate-based formulations would be less likely to provide sustained release from a thin (approximately 0.5 mm) film and that effort should be directed towards increasing the release of water-soluble small molecules from cholesterol and soybean oil-based formulations.

### Example 14-2: A semisolid lipid depot approach was utilized for controlled release of moxifloxacin hydrochloride from a thin layer.

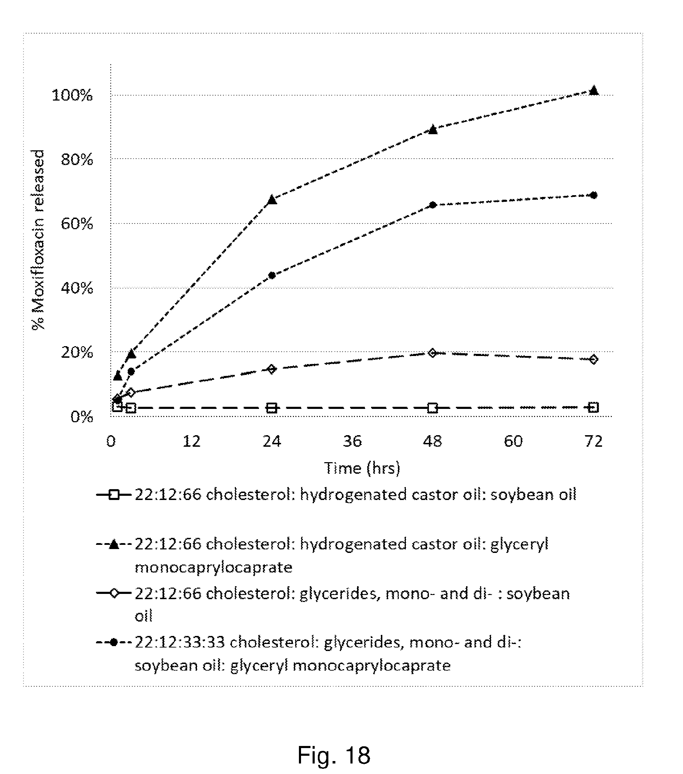

Four formulations based on cholesterol and soybean oil, shown in Fig. 18, and prepared with the method described above, were evaluated for in vitro elution per USP<1724> "Semisolid Drug Products - Performance Tests" using a Distek USP Type 2 dissolution apparatus and Distek Model B immersion cell with 100 mesh stainless steel membrane. In this and all subsequent examples, the samples were evaluated in a 200 mL vessel with 180 mL PBS at 32°C with 50 RPM stir rate, and the sample chamber was modified to 0.5 mm deep by 15 mm diameter by replacing the standard immersion cell sample chamber with a machined PTFE insert. The change in thickness was made so that the sample geometry reflected the expected product film thickness in use. With this change, cumulative release curve relevance to expected product performance was improved.

This designed experiment fixed the cholesterol and viscosity modifier concentrations at 22% and 12% and varied the composition of the oil phase and the identity of the viscosity modifier. The soybean oil-based formulation (22:12:66 cholesterol: hydrogenated castor oil: soybean oil) released 3.0% moxifloxacin hydrochloride at 72 hours, which would be considered insufficient for local administration of antibiotics to an acute surgical or traumatic wound. Release could be enhanced by exchanging soybean oil for glyceryl monocaprylocaprate (101.6% at 72 hours). A smaller enhancement was produced by exchanging the hydrogenated castor oil for glyceryl mono- and distearates (BASF Kolliwax GMS II), resulting in 17.9% release at 72 hours. Exchanging the hydrogenated castor oil for glycerides, mono- and di- and substitution of 50% of the soybean oil with glyceryl monocaprylocaprate resulted in 68.9% release at 72 hours. The release rate of a water-soluble small molecule, moxifloxacin hydrochloride, could be modulated by the identity of solid components in the formulation and the solvent blend selection.

In certain formulations, it can be possible to enhance in vitro elution of moxifloxacin hydrochloride by modifying a cholesterol-hydrogenated castor oil-soybean oil formulation by substituting glyceryl monocaprylocaprate for soybean oil or substituting GMSII for hydrogenated castor oil. According to the data shown herein, release profiles meeting the desired outcomes were achieved by adjusting the glyceryl monocaprylocaprate:soybean oil ratio in cholesterol-hydrogenated castor oil formulations or the oleic acid:soybean oil ratio in cholesterol-GMSII formulations.

### Example 14-3 - Examples of varying controlled release rates of moxifloxacin hydrochloride from formulations with varying mass percentage of glyceryl monocaprylocaprate.

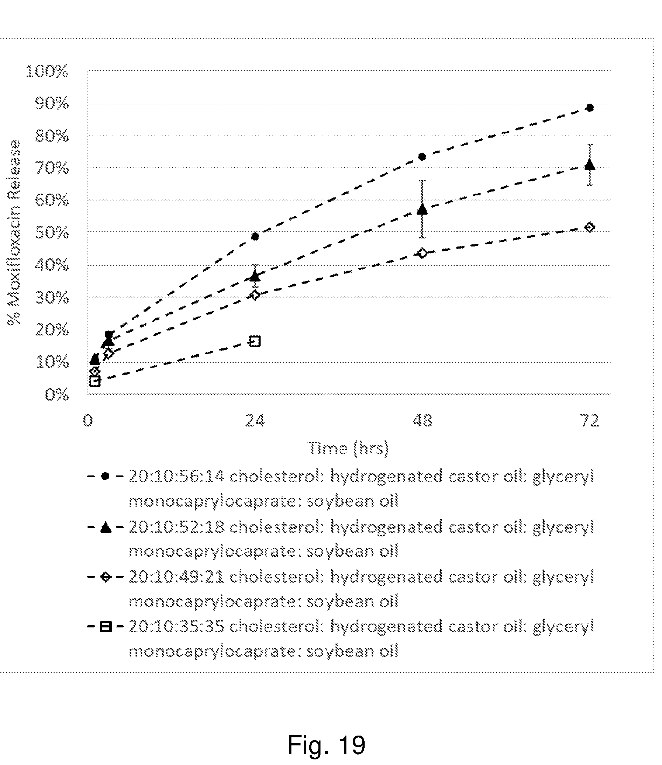

Four cholesterol-hydrogenated castor oil-glyceryl monocaprylocaprate-soybean oil formulations with varying solvent ratios, Fig. 19, were evaluated for in vitro elution per the above method. The formulation with the lowest glyceryl monocaprylocaprate content (20:10:35:35) was evaluated in run 1, formulations with 14% and 21% glyceryl monocaprylocaprate by mass in run 2 with identical IVE parameters. Six samples of formulation 20:10:52:18 cholesterol: hydrogenated castor oil: glyceryl monocaprylocaprate: soybean oil were evaluated in run 3 looking at the effect of aging the formulation on the in-vitro release of moxifloxacin hydrochloride. The formulation was prepared in a single batch, then remelted and allowed to age on the benchtop for 4 hours, 24 hours, 48 hours, 72 hours, 96 hours, and 168 hours prior to testing. The graph presented in Fig. 19 shows the combined results of that study as mean ± standard deviation excluding the data from the 4-hour aging condition. This short aging time was insufficient for the formulation to reach an equilibrium state, and all previously reported data was collected with formulation aged for a minimum of 18 hours.

The release rate of moxifloxacin hydrochloride can be selected by adjusting the ratio of glyceryl monocaprylocaprate to soybean oil, where greater glyceryl monocaprylocaprate content results in greater moxifloxacin hydrochloride release. For example, at 24 hours a 35:35 glyceryl monocaprylocaprate: soybean oil ratio resulted in 16.4% release, a 49:21 ratio produced 30.7% release, a 52:18 ratio produced 35.5% release, and a 56:14 ratio produced 48.9% release. A similar release optimization approach could be employed for other solid contents of semisolid ointment formulations. For this 20% cholesterol and 10% hydrogenated castor oil formulation family, 52% glyceryl monocaprylocaprate and 18% soybean oil is an exemplary formulation for release of moxifloxacin hydrochloride for an acute surgical or traumatic wound. The ratio of components for optimal release may be different for a different active pharmaceutical ingredient.

### Example 14-4 - Examples of varying controlled release rates of moxifloxacin hydrochloride from formulations with varying mass percentage of oleic acid.

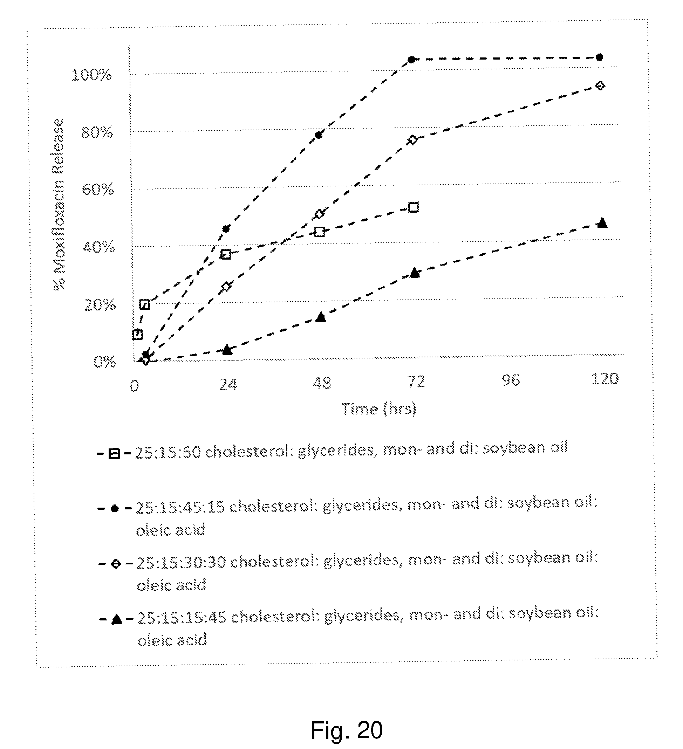

Four cholesterol-glycerides, mono- and di-soybean oil-oleic acid formulations with varying solvent ratios, Fig. 20, were evaluated for in vitro elution per the above method. The formulations containing oleic acid were evaluated in run 1, and formulation with soybean oil as the only solvent was evaluated in run 2 with identical IVE parameters.

The oleic acid-soybean oil ratio can be used to optimize moxifloxacin hydrochloride release rate with solids content of 25% cholesterol and 15% glycerides, mono- and di-. When soybean oil alone was used as a solvent, an initial burst was present (9.2% at 1 hour, 19.8% at 3 hours), with a slower sustained release rate. Addition of a minority of oleic acid (45:15 soybean oil: oleic acid) resulted in complete release at 72 hours (103%), while equal amounts (30:30) resulted in produced 77% moxifloxacin hydrochloride release, and the least release (29%) was observed with a majority of oleic acid (15:45 soybean oil: oleic acid). For this 25% cholesterol and 15% glycerides, mono- and di- formulation family, 30% soybean oil and 30% oleic acid is an exemplary formulation for release of moxifloxacin hydrochloride for an acute surgical or traumatic wound. The ratio of components for optimal release may be different for a different active pharmaceutical ingredient.

### Example 14-5: In vitro elution of moxifloxacin hydrochloride from exemplary formulations

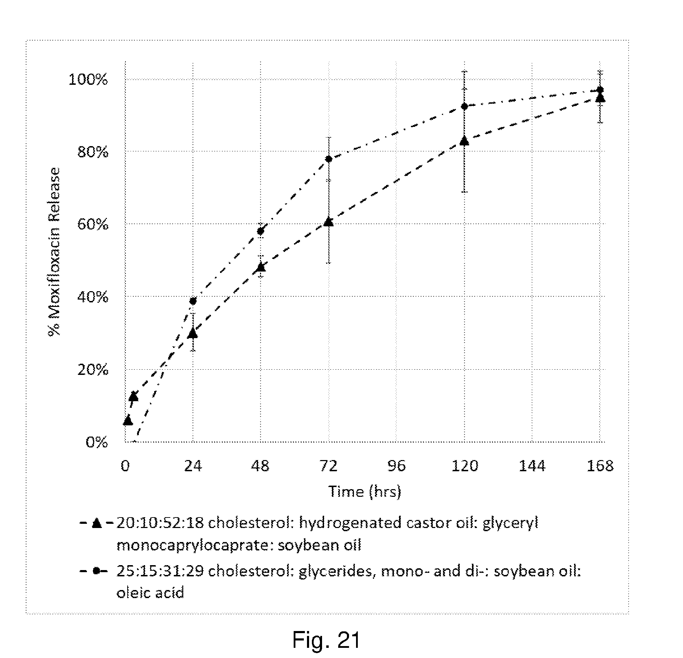

A preferred formulation family based upon cholesterol and hydrogenated castor oil in a blend of glyceryl monocaprylocaprate and soybean oil is described above. A formulation from this family, 20:10:52:18 cholesterol: hydrogenated castor oil: glyceryl monocaprylocaprate: soybean oil, was identified by its appropriate melting point in Table 4 and in vitro elution curve in Fig. 19. Another formulation was identified from a family containing cholesterol, glycerides, mono- and di-, soybean oil and oleic acid as shown in Fig. 20. Both of these exemplary formulations were prepared as described above with some additional steps appropriate in anticipation of possible future animal study preparation. The solid ingredients were equilibrated overnight in a dry nitrogen environment (< 10% RH) prior to addition of the oil components. Prior to dissolution or melting of components, the vial headspace was purged with nitrogen. Melting and dissolution was achieved at an oven setpoint temperature of 115 °C. These formulations were filled into a 4 mL syringe which was capped and placed in a nitrogen-purged foil pouch, and subsequently gamma irradiated at 28.0-34.4 kGy. The formulations were evaluated for in vitro elution per using the method above at the same custom 0.5mm thickness. Both formulations were evaluated in a single run with three replicates each. Results are displayed in Fig. 21as mean ± standard deviation. These elution profiles are appropriate for local delivery of moxifloxacin hydrochloride to an acute surgical or traumatic wound.

### Example 15 - Ex vivo application to simulated open wound site.

Simulated use testing was performed using beef or pork tissue with or without blood contamination. A series of formulations using various components described above were easy to apply and formed a robust visible coating on the tissue without blood contamination. In the following studies, a blood contamination step was added to the simulated use test. This was done by dipping the tissue in blood, then dipping it into saline and wiping the tissue with a gloved hand to simulate a battlefield wound irrigation. The blood-soaked and saline-rinsed simulated use handling was very selective and eliminated formulations based upon hydrogenated soy phosphatidylcholine and 12-hydroxystearic acid due to poor tissue spreading and irrigation resistance. Sodium stearate in glyceryl monocaprylocaprate and cholesterol in soybean oil plus a viscosity modifier were the primary families to emerge from initial studies; however, soybean oil-based formulations were challenging to apply to blood-contaminated tissue and did not demonstrate sufficient resistance to irrigation. Subsequent studies found that the addition of glyceryl monocaprylocaprate to cholesterol-soybean oil-based formulations significantly improved blood contaminated tissue handling and irrigation resistance. Oleic acid was shown to have a similar effect.

## Claims

1. An organogel composition for application to an open-wound site in a non-sterile environment comprising:
solid particles of a water-soluble antimicrobial or anti-infective agent; and,
an organogel matrix, wherein the solid particles are dispersed within the organogel matrix;
wherein the organogel matrix comprises a biocompatible organogelator and a biocompatible organic solvent, the organogel matrix having a melting point peak as measured by differential scanning calorimetry in the range of about 47° C to about 102° C,
wherein the biocompatible organogelator includes a mixture of cholesterol and at least one of hydrogenated castor oil or a mono- di- glyceride blend, and
wherein the biocompatible organic solvent includes soybean oil.

2. The organogel composition of claim 1, wherein the melting point peak is in the range of about 63° C to about 102° C.

3. The organogel composition of claim 1, wherein the melting point peak is in the range of about 47° C to about 66° C.

4. The organogel composition of any one of the preceding claims, wherein the organogel matrix defines a semi-solid state between the temperatures of about 0° C to about 70° C.

5. The organogel composition of claim 4, wherein the organogel matrix defines a semi-solid state between the temperatures of 4° C to about 65° C.

6. The organogel composition of any one of the preceding claims, wherein the organogelator further includes mono- or di- glycerides having a chain length greater than c10, hydrogenated oils, sodium salts of fatty acids, and combinations thereof.

7. The organogel composition of any one of the preceding claims, wherein the organogelator comprises less than 50% by weight of the organogel matrix.

8. The organogel composition of claim 6, wherein the organic solvent further includes castor oil, cottonseed oil, medium-chain triglyceride, glyceryl monocaprylocaprate, oleic acid, or linoleic acid, or mixtures or blends thereof.

9. The organogel composition of claim 8, wherein the organic solvent further includes glyceryl monocaprylocaprate or oleic acid.

10. The organogel composition of any one of the preceding claims, wherein the organogel composition is adherent to deep tissues.

11. The organogel composition of any one of the preceding claims, wherein the organogel composition, at a thickness of approximately 0.5 mm, exhibits an in vitro elution (IVE) profile of the water-soluble antimicrobial or anti-infective agent from the organogel matrix in phosphate buffered saline of between approximately 20% to 100% over a time range of approximately 2 days to 7 days.

12. The organogel composition of claim 11, wherein the IVE is approximately 40% or less in the first 24 hours.

13. The organogel composition of claim 11, wherein the IVE is approximately 80% or greater after 5 days.

14. The organogel composition of claim 11, wherein the IVE is between approximately 10% and 50% per day for days 1 to 3.

## Patentansprüche

1. Organogelzusammensetzung zum Auftragen auf eine offene Wunde in einer nicht sterilen Umgebung, umfassend:
feste Partikel eines wasserlöslichen antimikrobiellen oder antiinfektiösen Mittels; und
eine Organogelmatrix, wobei die festen Partikel innerhalb der Organogelmatrix dispergiert sind;
wobei die Organogelmatrix einen biokompatiblen Organogelbildner und ein biokompatibles organisches Lösungsmittel umfasst und die Organogelmatrix einen Schmelzpunktpeak, gemessen durch Differentialscanningkalorimetrie, im Bereich von etwa 47 °C bis etwa 102 °C aufweist,
wobei der biokompatible Organogelbildner eine Mischung aus Cholesterin und mindestens einem von gehärtetem Rizinusöl oder einer Mono-/DiglyceridMischung einschließt und
wobei das biokompatible organische Lösungsmittel Sojaöl einschließt.

2. Organogelzusammensetzung nach Anspruch 1, wobei der Schmelzpunktpeak im Bereich von etwa 63 °C bis etwa 102 °C liegt.

3. Organogelzusammensetzung nach Anspruch 1, wobei der Schmelzpunktpeak im Bereich von etwa 47 °C bis etwa 66 °C liegt.

4. Organogelzusammensetzung nach einem der vorstehenden Ansprüche, wobei die Organogelmatrix zwischen den Temperaturen von etwa 0 °C bis etwa 70 °C einen halbfesten Zustand definiert.

5. Organogelzusammensetzung nach Anspruch 4, wobei die Organogelmatrix zwischen den Temperaturen von 4 °C bis etwa 65 °C einen halbfesten Zustand definiert.

6. Organogelzusammensetzung nach einem der vorstehenden Ansprüche, wobei der Organogelbildner ferner Mono- oder Diglyceride mit einer Kettenlänge von mehr als c10, gehärtete Öle, Natriumsalze von Fettsäuren und Kombinationen davon einschließt.

7. Organogelzusammensetzung nach einem der vorstehenden Ansprüche, wobei der Organogelbildner weniger als 50 Gew.-% der Organogelmatrix umfasst.

8. Organogelzusammensetzung nach Anspruch 6, wobei das organische Lösungsmittel ferner Rizinusöl, Baumwollsamenöl, mittelkettiges Triglycerid, Glycerylmonocaprylocaprat, Ölsäure oder Linolsäure oder Mischungen oder Gemische davon einschließt.

9. Organogelzusammensetzung nach Anspruch 8, wobei das organische Lösungsmittel ferner Glycerylmonocaprylocaprat oder Ölsäure einschließt.

10. Organogelzusammensetzung nach einem der vorstehenden Ansprüche, wobei die Organogelzusammensetzung an tiefem Gewebe haftet.

11. Organogelzusammensetzung nach einem der vorstehenden Ansprüche, wobei die Organogelzusammensetzung bei einer Dicke von etwa 0,5 mm ein Profil der In-vitro-Elution (IVE) des wasserlöslichen antimikrobiellen oder antiinfektiösen Mittels aus der Organogelmatrix in phosphatgepufferter Kochsalzlösung von etwa 20 % bis 100 % über einen Zeitraum von etwa 2 Tagen bis 7 Tagen aufweist.

12. Organogelzusammensetzung nach Anspruch 11, wobei die IVE in den ersten 24 Stunden etwa 40 % oder weniger beträgt.

13. Organogelzusammensetzung nach Anspruch 11, wobei die IVE nach 5 Tagen etwa 80 % oder mehr beträgt.

14. Organogelzusammensetzung nach Anspruch 11, wobei die IVE an den Tagen 1 bis 3 zwischen etwa 10 % und 50 % pro Tag liegt.

## Revendications

1. Composition d'organogel destinée à être appliquée sur un site de plaie ouverte dans un environnement non stérile comprenant :
des particules solides d'un agent antimicrobien ou anti-infectieux soluble dans l'eau ; et,
une matrice d'organogel, dans laquelle les particules solides sont dispersées dans la matrice d'organogel ;
dans laquelle la matrice d'organogel comprend un organogélateur biocompatible et un solvant organique biocompatible, la matrice d'organogel ayant un pic de point de fusion tel que mesuré par calorimétrie à balayage différentiel compris dans la plage allant d'environ 47 °C à environ 102 °C,
dans laquelle l'organogélateur biocompatible comporte un mélange de cholestérol et d'au moins l'un parmi une huile de ricin hydrogénée ou un mélange de monoglycérides et de diglycérides, et
dans laquelle le solvant organique biocompatible comporte de l'huile de soja.

2. Composition d'organogel selon la revendication 1, dans laquelle le pic de point de fusion est compris dans la plage allant d'environ 63 °C à environ 102 °C.

3. Composition d'organogel selon la revendication 1, dans laquelle le pic de point de fusion est compris dans la plage allant d'environ 47 °C à environ 66 °C.

4. Composition d'organogel selon l'une quelconque des revendications précédentes, dans laquelle la matrice d'organogel définit un état semi-solide entre les températures allant d'environ 0 °C à environ 70 °C.

5. Composition d'organogel selon la revendication 4, dans laquelle la matrice d'organogel définit un état semi-solide entre les températures allant de 4 °C à environ 65 °C.

6. Composition d'organogel selon l'une quelconque des revendications précédentes, dans laquelle l'organogélateur comporte en outre des monoglycérides ou diglycérides ayant une longueur de chaîne supérieure à c10, des huiles hydrogénées, des sels de sodium d'acides gras et des combinaisons de ceux-ci.

7. Composition d'organogel selon l'une quelconque des revendications précédentes, dans laquelle l'organogélateur comprend moins de 50 % en poids de la matrice d'organogel.

8. Composition d'organogel selon la revendication 6, dans laquelle le solvant organique comporte en outre de l'huile de ricin, de l'huile de coton, du triglycéride à chaîne moyenne, du monocaprylocaprate de glycéryle, de l'acide oléique ou de l'acide linoléique, ou des mélanges ou combinaisons de ceux-ci.

9. Composition d'organogel selon la revendication 8, dans laquelle le solvant organique comporte en outre du monocaprylocaprate de glycéryle ou de l'acide oléique.

10. Composition d'organogel selon l'une quelconque des revendications précédentes, dans laquelle la composition d'organogel adhère aux tissus profonds.

11. Composition d'organogel selon l'une quelconque des revendications précédentes, dans laquelle la composition d'organogel, à une épaisseur d'environ 0,5 mm, présente un profil d'élution in vitro (IVE) de l'agent antimicrobien ou anti-infectieux soluble dans l'eau de la matrice d'organogel à une solution saline tamponnée au phosphate allant d'environ 20 % à 100 % sur une période allant d'environ 2 jours à 7 jours.

12. Composition d'organogel selon la revendication 11, dans laquelle l'IVE est d'environ 40 % ou moins au cours des premières 24 heures.

13. Composition d'organogel selon la revendication 11, dans laquelle l'IVE est d'environ 80 % ou plus après 5 jours.

14. Composition d'organogel selon la revendication 11, dans laquelle l'IVE est compris entre environ 10 % et 50 % par jour pour les jours 1 à 3.
